(19) Europäisches Patentamt
European Patent Office
Office européen des brevets

(11) EP 2 735 314 B1

(12) **EUROPEAN PATENT SPECIFICATION**

(45) Date of publication and mention of the grant of the patent:
**17.10.2018 Bulletin 2018/42**

(21) Application number: **12779704.1**

(22) Date of filing: **01.05.2012**

(51) Int Cl.:
***A61K 39/39*** (2006.01) ***A61K 9/127*** (2006.01)
***A61K 31/7088*** (2006.01) ***A61K 38/00*** (2006.01)
***A61K 39/00*** (2006.01) ***A61K 47/36*** (2006.01)
***A61K 48/00*** (2006.01) ***A61P 35/00*** (2006.01)
***C07K 14/705*** (2006.01)

(86) International application number:
**PCT/JP2012/002958**

(87) International publication number:
**WO 2012/150663 (08.11.2012 Gazette 2012/45)**

(54) **SUGAR-COATED LIPOSOME COMPOSITION**

DRAGIERTE LIPOSOMENZUSAMMENSETZUNG

COMPOSITION DE LIPOSOMES ENROBÉS DE SUCRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.05.2011 JP 2011102962**

(43) Date of publication of application:
**28.05.2014 Bulletin 2014/22**

(73) Proprietors:
- **BioMedCore Inc.**
  **Kanagawa 230-0046 (JP)**
- **Sapporo Medical University**
  **Sapporo-shi, Hokkaido 060-0061 (JP)**
- **Tokai University Educational System**
  **Tokyo 151-0063 (JP)**

(72) Inventors:
- **KAJIWARA, Toshimitsu**
  **Hokkaido 064-0806 (JP)**
- **NUMAZAKI, Maki**
  **Naka-gun**
  **Kanagawa 259-0133 (JP)**
- **SHIMIZU, Yoshitaka**
  **Hokkaido 062-0936 (JP)**
- **TAMURA, Yasuaki**
  **Sapporo-shi**
  **Hokkaido 060-8556 (JP)**
- **KOJIMA, Naoya**
  **Hiratsuka-shi**
  **Kanagawa 259-1292 (JP)**

(74) Representative: **J A Kemp**
**14 South Square**
**Gray's Inn**
**London WC1R 5JJ (GB)**

(56) References cited:
**EP-A1- 1 870 091** **WO-A1-2005/087196**
**WO-A1-2006/104199** **WO-A1-2010/032408**
**WO-A1-2011/024482** **JP-A- 2007 308 472**

- **THOMANN JEAN-SÉBASTIEN ET AL: "Antitumor activity of liposomal ErbB2/HER2 epitope peptide-based vaccine constructs incorporating TLR agonists and mannose receptor targeting.", BIOMATERIALS JUL 2011, vol. 32, no. 20, July 2011 (2011-07), pages 4574-4583, XP002739915, ISSN: 1878-5905**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 735 314 B1

SEQUENCE LISTING

<110> BIOMEDCORE Tokai University Educational System Sapporo Medical University

<120> Modified Liposome Composition

<130> PW12008BM

<150> JP2011-102962
<151> 2011-05-02

<160> 2

<170> Patent In version 3.1

<210> 1
<211> 27

<212> DNA
<213> Homo sapiens

<400> 1
gcttacgcct gtaataccag cactttg 27

<210> 2
<211> 9
<212> PRT
<213> Homo sapiens

<400> 2

```
Ala Tyr Ala Cys Asn Thr Ser Thr Leu
1               5
```

**Description**

Technical Field

The present invention relates to a sugar-coated liposome to be used as a vaccine or an adjuvant, and to use of said liposome. Particularly, the present invention relates to a mannose-coated liposome to be used as a vaccine or an adjuvant in a cancer treatment and use thereof.

Background Art

In a healthy body, the immune system usually has mechanisms to recognize and damage cancer cells, which suppresses emergence and proliferation of cancer cells. A main mechanism therefor is cell-mediated immunity mediated by cytotoxic T lymphocytes (CTLs), natural killer cells and the like. Among these cells, antigen-specific CTLs are considered to be main effector cells in the mechanisms for removing cancer cells. Cancer vaccines are vaccines administered for the purpose of inducing activation of said antigen-specific CTL, and are expected as new therapeutic tools for many intractable cancers. Cancer antigens to be used in cancer vaccines include many antigens such as CEA, survivin 2B, MUC-1, and WT-1 (Non-Patent Literature 1).

Peptide vaccines are **phagocytized** by antigen presenting cells (dendritic cells, macrophages and the like) in the body, and antigens are presented by major histocompatibility complex (MHC) molecules. The CD8+T cells recognize the antigens presented by MHC class I molecules, and are then activated, differentiated and matured into antigen-specific CTLs. It is considered that the activation of CD8+ cells usually requires a help such as MHC class II and stimulations by cytokines (IL-2, IL-12 and the like) which are released from CD4+T cells (Th cells) activated by the epitopes presented by said MHC class II (Non-Patent Literature 1). In particular, it is known that the proliferation of CTLs requires co-stimulation with IL-2. Indeed, it is reported that single use of an MHC class I epitope antigen cannot kill many cancer cells in the body because of no assistance from Th cells and of weak production of IL-2 from CD8+CTLs (Non-Patent Literature 2). It is reported that such help by CD4+T cells is required, in particular, for induction of CTLs using a minor histocompatibility antigen, a tumor antigen, a soluble protein, a synthetic peptide, dendritic cells pulsed with a peptide, and for induction of CTLs in the infection of virus, such as herpes simplex virus and influenza.

It is also known that tumors have mechanisms to suppress the function of immunity. For example, cytokines, such as IL-10, produced by tumors and their stroma cells are known to induce cells that promote immunosuppression and immune tolerance, such as CD4+CD25+Foxp3+ regulatory T ($T_{reg}$) cells, myeloid derived suppressor cells, and tolerogenic dendritic cells, to induce anergy in effector T cells, and to modulate intratumoral immune environment toward Th2-predominant, thereby negatively control tumor immune response. Therefore, it has been reported that induction or introduction of tumor destructive CD8+T cells alone leads to insufficient induction and activation of antitumor immunity in tumor vaccine therapy and the cancer cell therapy (Non-Patent Literature 4). In fact, a clinical trial using the survivin 2B peptide antigen (0.1, 1.0 or 10.0 mg) failed to demonstrate a therapeutic effect (Non-Patent Literature 5). Also, only 2.9% of efficacy rate was obtained in a clinical trial in which 323 metastatic cancer patients were given 1 mg of cancer vaccine containing an antigenic peptide (MHC class I antigen) recognized by CD8+ cells every three weeks (Non-Patent Literature 6).

For the purpose of solving such problems and make the cancer immunotherapy practical, adjuvants and techniques to release from immunosuppression mechanism have been developed. For example, efforts to activate CTLs have been made by using MHC class I antigens in combination with adjuvants. In one case, a therapeutic effect was observed in use of Montanide ISA51 adjuvant with 3 mg of Wilms tumor gene antigen peptide every week for three months (Non-Patent Literature 7, Non-Patent Literature 8). Other known adjuvants that induce the cell-mediated immunity include heat shock proteins (HSPs), and HSP70, HSP90, gp96 and the like have been used. HSPs are taken up by antigen presenting cells and promote cross-presentation of other antigen molecules. For example, efforts to increase the antitumor effect have been made by using survivin 2B antigen peptide with HSP90 or HSP70 (Non-Patent Literature 9).

Ligands for Toll-like receptor (TLR) 3 and TLR9 have been also reported to induce CTLs without help of CD4+T cells and stimulate the proliferation of CTLs. For example, administration of 10 to 30 $\mu$g of the CTL peptide consisting of 257th to 264th of the amino acid sequence of ovalbumin (OVA) in combination with 25 $\mu$g of poly (I:C) or CpG-B to mice has been reported to not only induce antigen-specific CTLs and also promote the proliferation of CTLs. It has been disclosed that this CD4+T cell independent CTL induction does related neither to increased expression of MHC class I molecules or CD86 in dendritic cells nor to the promotion of production of IL-12, but relates to interferon $\alpha/\beta$ production (Non-Patent Literature 3). There are also others reporting that IL-12 and interferon $\gamma$ (IFN-$\gamma$) are not necessary for the induction of CTL response and, in particular, CTL exerts anticancer activity even in the absence of IL-12 (Non-Patent Literature 10).

As an example of using cancer vaccine therapy in combination with release from the immunosuppression mechanism, it is reported that median survival rate was elongated in 171 patients with human non-small-cell lung cancer

who were administered cyclophosphamide (300 mg/$m^2$) to release immune tolerance and then administered MUC-1 antigen BLP25 (1 mg) encapsulated in liposome in combination with adjuvant (monophosphoryl lipid A) every six weeks for five months(Non-Patent Literature 11).

Despite of their merits, these adjuvants and release from the immunosuppression mechanism are not sufficient to obtain enough antitumor effects in using CTL antigens as vaccines in the cancer immunotherapy. Accordingly, superior formulations have been desired for practical use of CTL antigens as vaccines for the cancer immunotherapy.

Taking advantage of macrophage's recognition of bacteria via sugar chain recognizing receptors (such as macrophage mannose receptor CD206), the vaccine delivery via the sugar chain recognizing receptors directed to dendritic cells and macrophages has been proposed. Liposomes having mannose conjugated on their surface are known to transport encapsulated vaccines to dendritic cells and macrophages as well as to have an adjuvant activity for inducing the cell-mediated immunity (Patent Literature 1, Non-Patent Literature 12). In particular, mannose-coated liposomes are considered to promote the antigen presentation with MHC class II molecules as well as MHC class I molecules, to induce production of Th1 cytokines such as IFN-γ and IL-12, and to promote the activation of CTLs (Patent Literature 2).

For example, it has been reported that mannotriose-coated liposomes (Man3-Lipo) are taken up quickly by peritoneal macrophages (PEMs), which leads said macrophages to mature, to express the costimulators CD40, CD80, CD86 and MHC class II molecules on their surface, and to produce IL-12. It is also reported that this activation of macrophages by Man3-Lipo does not involve production of IL-1 and IL-6, and thus is different from the response by TLR ligands (Non-Patent Literature 13).

Murine splenocytes sensitized with OVA encapsulated in Man3-Lipo have been reported to produce the Th1 cytokine IFN-γ upon resensitization with OVA (Non-Patent Literature 13). Similarly, murine splenocytes sensitized with a leishmania antigen encapsulated in Man3-Lipo and Man5-Lipo have been reported to produce the antigen-specific Th1 cytokine IFN-γ upon resensitization with the antigen, and that its productions of the Th2 cytokines IL-4 and IL-5 are suppressed (Non-Patent Literature 14).

## Citation List

### Patent Literature

Patent Literature 1: Japanese Patent Laid-Open No. 7-126185
Patent Literature 2: International publication No. WO 2006/104199


### Non-Patent Literature

Non-Patent Literature 1: Iwayama et al., Gekachiryo ("Surgical Treatment" in Japanese), Vol.96, pp.50-54 (2007)
Non-Patent Literature 2: Takashi Nishimura, Igakunoayumi ("Proceedings in Medicine" in Japanese), Vol.221, No. 8, pp.641-646
Non-Patent Literature 3: Sandra Hervas-Stubbs et al., BLOOD, Vol.109 5318-5326 (2007)
Non-Patent Literature 4: Ikeda et al., Experimental Medicine, Vol.27, No.14, 2176-2184 (2009)
Non-Patent Literature 5: Tsuruma Tetsuhiro et al., Japanese Journal of Cancer and Chemotherapy, vol.31, No.11, 1634-1636 (2004)
Non-Patent Literature 6: Rosenberg, S.A. et al., Nature Medicine, Vol.10, 909-915 (2004)
Non-Patent Literature 7: Oka et al., Japanese Journal of Clinical Immunology, Vol.31, No.5, 375-382 (2008)
Non-Patent Literature 8: Tsuboi, A et al., Int. J. Hematol., Vol.86, 414-417 (2007)
Non-Patent Literature 9: Kurotani et al., The Journal of Immunology, Vol.179, pp.1803-1813
Non-Patent Literature 10: Lakshmi Krishnan et al., Cancer Research, Vol.63, 2526-2534 (2003)
Non-Patent Literature 11: Sangha R. et al., Clin. Cancer Res., 13 (15 Pt2), s4652-4654 (2007)
Non-Patent Literature 12: Fukusawa et al, FEBS Letters, Vol.441, pp.353-356 (1998)
Non-Patent Literature 13: Takagi et al, Cytokine, vol.40, pp.241-250 (2007)
Non-Patent Literature 14: Y. Shimizu et al, Parasite Immunology, Vol.29, 229-239 (2007)


## Summary of Invention

Accordingly, an object of the present invention is to provide a formulation that is more effective in promoting cell-mediated immunity as an adjuvant. As described above, it has been already known that sugar-coated liposome

promotes the antigen presentation by both of the MHC class I and MHC class II and induces production of Th1 cytokines such as IFN-γ and IL-12. However, as described above, IFN-γ and IL-12 are known not to be involved in CD4+ independent CTL induction using MHC class I epitope alone. Thus, the ability of sugar-coated liposomes to induce the CTL in using an MHC class I epitope alone (in the absence of an MHC class II epitope) was totally unknown. In particular, it is considered to be impossible to induce CD4+ independent CTL via TLR as described above, since sugar is not a ligand of TLR3 or TLR9.

In an aspect of practical use of vaccine, in order to secure a stable supply of the vaccine, it is preferable that a small amount of antigens exert a sufficient effect. As described above, the amount of antigen currently used in the cancer immunotherapy is, for example, about 0.1 to 10 mg for human, and several tens of micrograms for mice. A cancer vaccine formulation effective with less amount of antigen has been desired.

The inventors have studied on promotion of cell-mediated immunity using sugar-coated liposomes and achieved to activate CTL independently of MHC class II molecules (independent of CD4+T cells) by administering sugar-coated liposome (in particular mannose-coated liposome) with an antigen. The inventors also investigated on the amount of antigen peptide vaccine required for activation of cell-mediated immunity using a mouse model and found that the sugar-coated liposome of the present invention can achieve a sufficient therapeutic effect against cancer with only an amount of 0.1 to 10 ng of antigen (corresponding to about 0.5 ng to 30 μg of antigen for human).

Sugar-coated liposomes conventionally used are sized with an approximately 1 μm filter (See, for example, Naoya Kojima et al., Journal of Controlled Release, Vol.129:pp.26-32 (2008); Takagi et al., Cytokine, Vol.40, pp.241-250 (2007); Y. Shimizu et al., Parasite Immunology, Vol.29; Yuzuru Ikehara et al., Cancer Res., Vol.66, No. 17: pp.8740-8748 (2006)). However, as the particle diameter of liposome increases, aggregation tendency is expected to increase. In addition, a very small dose would lead difficulty in maintaining uniformity of content volume (dose). Furthermore, when lyophilized preparation is required to increase storage stability of a liposome formulation, particle diameter distributions of liposome with a large particle diameter may greatly change after lyophilization, that would bring a problem in control of particle size. Accordingly, a highly effective liposome with smaller particle diameter is desired. The inventors have studied on the particle size of sugar-coated liposome and found that the particle size of sugar-coated liposome correlates with adjuvant activity thereof. In particular, the inventors found that sugar-coated liposomes with particle size equal to or more than 100 nm markedly increase IL-12 production and decrease IL-6 production and thereby has a high ability to induce an immune response toward to cell-mediated immunity. The inventors have also found that sugar-coated liposomes with particle sizes of at least 1,000 nm have sufficient ability to induce an immune response in cell-mediated immunity.

Relative amounts of sugars in conventional sugar-coated liposomes was DPPC : cholesterol : sugar lipid = 1:1:0.1 (See, for example, Y. Shimizu et al., Parasite Immunology, Vol. 29: 229-239 (2007); Yuzuru Ikehara et al., Cancer Res., Vol. 66, No. 17 pp.8740-8748 (2006)). The inventors have studied on relation between relative amounts of sugars to coat sugar-coated liposomes and their activities and found that liposomes with a ratio of glycosylated to non-glycosylated lipid equal to or higher than 0.00075 have high ability to induce CTLs.

Accordingly, the present invention relates to a mannose-coated liposome which can activate CTLs to promote cell-mediated immunity with a small amount of antigen. Specifically, the present invention provides the following (1) to (14):

(1) A mannose-coated liposome with a molar ratio of glycosylated to non-glycosylated lipid of 0.00075 or more and a particle size of 180 to 1100 nm for use in a method of treatment, wherein the method comprises administering the mannose-coated liposome and an MHC class I molecule-binding peptide.

(2) The mannose-coated liposome for use of (1), wherein:

- the MHC class I molecule-binding peptide is encapsulated in the mannose-coated liposome; or
- the mannose-coated liposome is separately administered at the same time, at intervals, or continuously with the MHC class I molecule-binding peptide.

(3) The mannose-coated liposome for use of (1) or (2), wherein

- said mannose-coated liposome can induce and/or lead proliferation of an antigen-specific CTL independently of an MHC class II molecule; and/or
- the MHC class I molecule-binding peptide is an antigen which cannot induce and/or lead proliferation of an antigen-specific CTL in the absence of an MHC class II antigen.

(4) The mannose-coated liposome for use of (1), wherein the MHC class I molecule-binding peptide is a tumor specific antigen, a soluble protein, a virus antigen, a protozoan antigen, a fungal antigen, or a bacteria antigen.

(5) The mannose-coated liposome for use of (1), wherein the MHC class I molecule-binding peptide is a tumor antigen.

(6) The mannose-coated liposome for use of (5), wherein the tumor antigen is a peptide antigen comprising an

amino acid sequence from 80th to 88th of survivin 2B.

(7) The mannose-coated liposome for use of any one of (1) - (6), wherein the molar ratio of glycosylated to non-glycosylated lipid is:

- 0.00075 to 0.075;
- 0.0075 or more; or
- 0.0075 to 0.075.

(8) The mannose-coated liposome for use of any one of (1)-(7), wherein the sugar is oligomannose.

(9) The mannose-coated liposome for use of (8), wherein the oligomannose is mannobiose, mannotriose, mannotetraose, mannopentaose, mannohexaose or mannoheptaose.

(10) A mannose-coated liposome, wherein:

- a particle size of the mannose-coated liposome is 180 to 1100 nm; and
- a molar ratio of glycosylated to non-glycosylated lipid is:
- 0.00075 or more;
- 0.00075 to 0.075;
- 0.0075 or more; or
- 0.0075 to 0.075.

(11) The mannose-coated liposome of (10), wherein the sugar is oligomannose.

(12) The mannose-coated liposome of (11), wherein the oligomannose is mannobiose, mannotriose, mannotetraose, mannopentaose, mannohexaose or mannoheptaose.

(13) A pharmaceutical composition comprising a liposome as defined in any one of (1) to (12) as an active ingredient, for use in a method of treatment which is an immunotherapy for cancer.

(14) The pharmaceutical composition for use according to (13), wherein the pharmaceutical composition is administered in a dose of 0.5 ng to 30 $\mu$g per patient.

As used herein, "MHC class I molecule-binding peptide" refers to a peptide which is taken up by an antigen presenting cell, and then presented by MHC class I molecules. Whether a certain peptide is an MHC class I molecule-binding peptide or not can be determined, for example, by having antigen presenting cells take up a peptide fragment of interest, bringing them in contact with T cells, and then detecting produced cytokines or activated CTLs as an indicator. The peptide is an MHC class I molecule-binding peptide, if Th1 cytokines such as IFN-$\gamma$ or IL-12 are produced and/or CTL recognizing the peptide is activated. An MHC class I molecule-binding peptide can be obtained by peptide mapping: an MHC class I molecule-binding peptide can be obtained by having antigen presenting cells take up a peptide fragment that is expected to be cleaved by proteasome, bringing them in contact with T cells, then using produced cytokines or activated CTLs as an indicator, and selecting 7 to 10 amino acids that ultimately induce production of Th1 cytokines such as IFN-$\gamma$ and IL-12, and/or activate CTLs recognizing the peptide.

As used herein, "nucleic acid encoding an MHC class I molecule-binding peptide" refers to a nucleic acid that produces an MHC class I molecule-binding peptide by translation in a living body. The nucleic acid may have a nucleic acid encoding an MHC class I molecule-binding peptide, as well as regions necessary for transcription and translation of said nucleic acid. Preferably, the nucleic acid encoding an MHC class I molecule-binding peptide is a nucleic acid that is taken up by antigen presenting cells, and produces the MHC class I molecule-binding peptide in the cells.

The MHC class I molecules preferably match with the alleles (HLA haplotype) of the human leukocyte antigen (HLA) of the subject for administration. Thus, it is preferable to use an HLA haplotype of the subject of administration restricted MHC class I molecule-binding peptide or a nucleic acid encoding said peptide. The MHC class I molecule-binding peptide used herein may include naturally occurring amino acids, as well as artificially modified amino acids. For example, the number of amino acids of the MHC class I molecule-binding peptide is preferably 7 to 10 amino acids. For example, the MHC class I molecule-binding peptide may be a peptide having a part of an amino acid sequence of a minor histocompatibility antigen; a tumor specific antigen; a soluble protein; an antigen of virus such as herpes simplex virus and influenza virus; an antigen of parasite such as leishmania, toxoplasma, and malaria; an antigen of fungus such as candida, aspergillus, and cryptococcus; or an antigen of bacterium such as mycoplasma, streptococcus, that can bind to an MHC class I molecule. Examples of the tumor specific antigen include antigens derived from proteins of tumor viruses such as Hepatitis B virus, SV40/ polyoma virus, humans papillomavirus (e.g., E6 and E7 proteins), human adenovirus type 5, humans herpesvirus type 8, Shope fibroma virus, Rous sarcoma virus, and human T cell leukemia virus; and antigens derived from proteins specifically expressed in cancer cells such as ras, p53, survivin 2B, CEA, MUC-1, and WT-1. Examples of the antigen derived from survivin 2B include peptides composed of the amino acid sequence of the survivin 2B80-88 peptide antigen (Ala-Tyr-Ala-Cys-Asn-Thr-Ser-Thr-Leu: SEQ ID NO: 2). This peptide

is particularly effective for patients with the HLA haplotype of HLA-A24. The nucleic acid encoding an MHC class I molecule-binding peptide includes a nucleic acid encoding peptide having a part or all of the amino acid sequence of a minor histocompatibility antigen, a tumor specific antigen (hepatitis B virus, SV40 / polyoma virus, human papillomavirus (e.g., E6 and E7 proteins), an antigen derived from proteins of tumor virus such as human adenovirus type 5, human herpes virus type 8, Shope fibroma virus, Rous sarcoma virus, human T cell leukemia virus; an antigen derived from proteins specifically expressed in cancer cells such as ras, p53, survivin 2B, CEA, MUC-1, WT-1); a soluble protein, an antigen of virus such as herpes simplex virus, and influenza virus; an antigen of parasitic such as leishmania, toxoplasma, and malaria; an antigen of fungus such as candida, aspergillus, and cryptococcus; an antigen of bacterium such as mycoplasma and streptococcus; and being capable of binding to an MHC class I molecule. For example, if the nucleic acid encoding an MHC class I molecule-binding peptide encodes the survivin 2B80-88 peptide, the sequence may be GCTTACGCCTGTAATACCAGCACTTTG (SEQ ID NO: 1) .

The MHC class I molecule-binding peptide may be a peptide in which an amino acid is added / inserted to or an amino acid is substituted in an amino acid sequence derived from a naturally occurring antigen such as a minor histocompatibility antigen; a tumor specific antigen; a soluble protein; an antigen of virus such as herpes simplex virus, and influenza virus; an antigen of parasitic such as leishmania, toxoplasma, and malaria; an antigen of fungus such as candida, aspergillus, and cryptococcus; an antigen of bacterium such as mycoplasma and streptococcus. The amino acids composing the MHC class I molecule-binding peptide may be modified at an end or inside. The MHC class I molecule-binding peptide may be conjugated with a stabilizing substance such as PEG at an end or an internal amino acid, or may have two or more peptides connected through a linker and the like, or may form a dimer. For example, the survivin 2B80-88 peptide can form a dimer via cysteine residues in the peptide. Such a survivin 2B80-88 peptide dimer is included in the MHC class I molecule-binding peptide disclosed herein.

As used herein, "an antigen which cannot induce and/or lead proliferation of an antigen-specific CTL in the absence of an MHC class II antigen" means an MHC class I molecule-binding peptide that cannot induce and/or lead proliferation of an antigen-specific CTL to a degree needed to have a therapeutic effect under an environment without stimulation of MHC class II molecules. Whether a certain antigen can induce and/or lead proliferation of an antigen-specific CTL in the absence of MHC class II antigens or not can be determined by administering a candidate antigen peptide to a disease model animal sensitive to said antigen without administering an MHC class II molecule-binding peptide or an MHC class II molecule stimulating substance, and after a desired period of time to induce an immune response, by evaluating the degree of cure of the disease. The evaluation of the degree of cure can be made as follows: comparing the candidate peptide administered animals with control animals to which the peptide is not administered, and determining that in the case that single administration of the peptide cannot exert sufficient therapeutic effect against control and thus the peptide is considered not to be useful as a pharmaceutical product, then the peptide is evaluated to be a peptide that cannot induce and/or lead proliferation of an antigen-specific CTL to a degree needed to have a therapeutic effect under an environment without stimulation of MHC class II molecules.

As used herein, "sugar-coated liposome" refers to a liposome that has at least one sugar conjugated to the surface thereof. The sugar-coated liposome may have other substance conjugated thereto, as far as a sugar is bound to the surface thereof. As used herein, a sugar-coated liposome "for use with an MHC class I molecule-binding peptide or a nucleic acid encoding said peptide" means a sugar-coated liposome in which an MHC class I molecule-binding peptide or a nucleic acid encoding said peptide is encapsulated, or alternatively a combination of separately prepared a sugar-coated liposome and an MHC class I molecule-binding peptide or a nucleic acid encoding said peptide to be use together. The present disclosure also encompasses a sugar-coated liposome that is specifically prepared for use with an MHC class I molecule-binding peptide or a nucleic acid encoding said peptide. When a sugar-coated liposome and an MHC class I molecule-binding peptide or a nucleic acid encoding said peptide is provided in separate formulations, the sugar-coated liposome may be a sugar-coated liposome which is separately administered at the same time with, at intervals, or continuously with an MHC class I molecule-binding peptide or a nucleic acid encoding the peptide. The adjuvant effect of the sugar-coated liposome is similarly exerted either when administered in a state encapsulating an antigen or when administered separately from the antigen.

The sugar-coated liposome of the present invention is a mannose-coated liposome.

The sugar may be a monomer or a polymer of plural sugars (no matter if it is linear or branched), and the number of sugar in such a polymer is 2 to 11. For example, mannobiose, mannotriose, mannotetraose, mannopentaose, mannohexaose or mannoheptaose may be used. Two or more sugars can be linked with $\alpha$1-2 bond, $\alpha$1-3 bond, $\alpha$1-4 bond, $\alpha$1-6 bond, $\beta$1-4 bond or a combination thereof. When the sugar contained in the sugar-coated liposome is a polymer of plural sugars, the polymer may include a component (e.g., a constituent sugar) that does not bind to lectins of antigen presenting cells, as far as the polymer binds to a lectin of an antigen presenting cell. Whether a certain sugar can bind to a lectin of an antigen presenting cell can be determined by a method well known to those skilled in the art, for example, by adding a labeled sample sugar to a solid-phase having a lectin immobilized, and measuring the degree of binding of said sugar to said lectin by measuring said label. Preferably, the sugar contained in the sugar-coated liposome is a sugar that bind to a lectin with an avidity equal to or stronger than mannobiose, mannotriose, mannotetraose,

mannopentaose, mannohexaose or mannoheptaose.

For example, mannopentaose and mannoundecaose can have the following structures. In the following formulae, Man denotes mannose, and GlcNAc denotes N-acetylglucosamine. In Formula 2, Man (mannose) linked by α1-2 bond may each independently be present or absent.

[Formula 1]

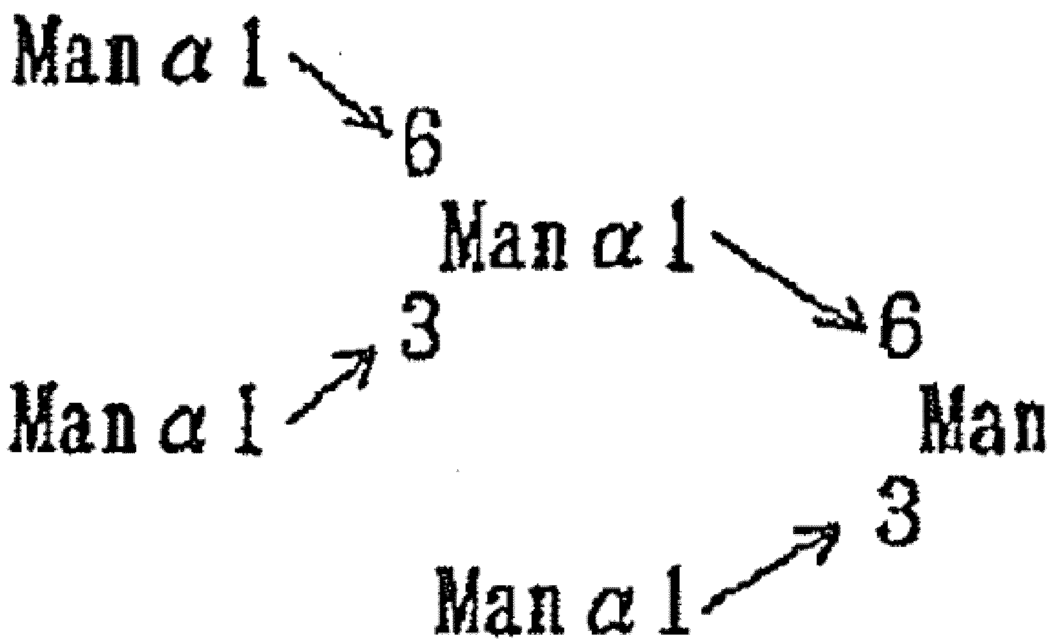

[Formula 2]

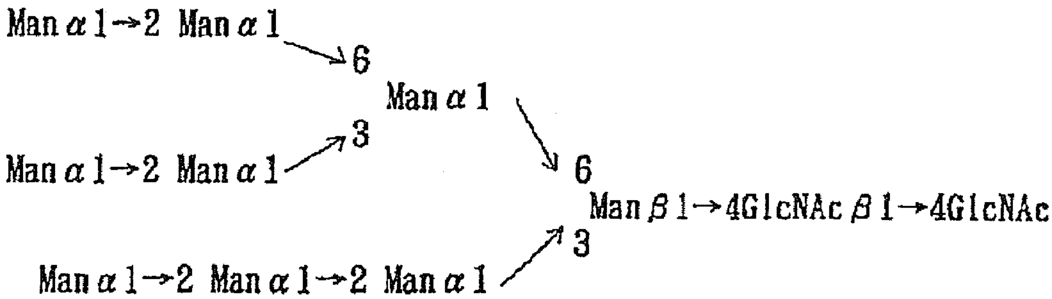

The mannose-coated liposome of the present invention is, preferably, a mannobiose-coated liposome, a mannotriose-coated liposome, a mannotetraose-coated liposome, a mannopentaose-coated liposome, a mannohexaose-coated liposome or a mannoheptaose-coated liposome, and more preferably, a mannotriose-coated liposome.

The relative amount of sugar that coats the sugar-coated liposome in a molar ratio to non-glycosylated lipid (glycosylated lipid / non-glycosylated lipid) is equal to or more than 0.00075; more preferably, equal to or more than 0.0025; and most preferably equal to or more than 0.0075. For example, the relative amount of sugar that coat the sugar-coated liposome in a molar ratio to non-glycosylated lipid (glycosylated lipid / non-glycosylated lipid) may be 0.00075 to 0.075, is preferably 0.0025 to 0.075, and more preferably 0.0075 to 0.025. The relative amount of sugar that coats the sugar-coated liposome are determined as appropriate by a method well known to those skilled in the art, preferably, quantified by a measuring method combining HPLC and evaporative light scattering detector (Shimizu, Y., J. et al., Chromatogr. B., 754:127-133 (2001)). More specifically, the measurement can be done using ELSD (brand name "SEDERE SEDEX-55") as a detector, Wakosil 5TMS (4.6 mm in diameter, 25 cm in length, particle size 5 μm; manufactured by Wako Pure Chemical Industries, Ltd.) as a column, Wakosil 5TMS (4.6 mm in diameter, 1 cm in length) as a guard column, chloroform / methanol / water (1:33:6 [volume ratio]) as an eluent, isocratic elution at 0.8 ml/min, and an injection

volume of 20 μL.

The particle size and reactivity of the sugar-coated liposome of the present invention exhibit a bell-shaped correlation. Accordingly, particles with suitable reactivity can be provided by making particles with an appropriate size range. The lower limit of particle size is 180 nm. The upper limit of particle size of the sugar-coated liposome may be 1100 nm, 1000 nm, 900 nm, 800 nm, 700 nm, 600 nm, 550 nm, 500 nm. For example, the particle size of the sugar-coated liposome may be 180 to 700 nm, 200 to 700 nm, 300 to 700 nm, 300 to 600 nm, 400 to 600 nm or 450 to 600 nm. Alternatively, the particle size of the sugar-coated liposome may be 180 to 1100 nm, 250 to 1100 nm, 300 to 1100 nm, 180 nm to 1000 nm, 180 nm to 800 nm, 250 to 800 nm, 300 to 800 nm, 180 nm to 500 nm, 250 to 500 nm, 300 to 500 nm. As used herein, the particle size of the sugar-coated liposome refers, unless otherwise mentioned, to a particle size determined by the following method: diluting the prepared sugar-coated liposome with PBS, subjecting it measurement with a particle size analyzer (manufactured by Malvern Instruments Ltd., ZETA SIZER Nano-ZS) by dynamic light scattering, and calculating the particle size with the program attached to the measuring equipment. When the sugar-coated liposome of the present invention is defined by a specific particle size (e.g., being sugar-coated liposomes with a particle size of 300 to 600 nm), the liposome may contain liposomes with other particle sizes, as far as the liposome having the particle size (e.g., sugar-coated liposomes with a particle size of 300 to 600 nm) is included as a main sugar-coated liposome. "Included as a main sugar-coated liposome" may mean that for example, equal to or more than 30%, equal to or more than 40%, equal to or more than 50%, equal to or more than 60%, equal to or more than 70%, equal to or more than 80%, equal to or more than 90% of the sugar-coated liposome is included within the particle size range of the sugar-coated liposome of the present invention. Preferably, when the sugar-coated liposome of the present invention is defined by a specific particle size, the particle size refers to a mean particle size of the sugar-coated liposome.

As used herein, "liposome" refers to a vesicle formed by a lipid-bilayer membrane enclosing aqueous medium within, wherein the vesicle may have one or more layers of the lipid-bilayer membrane. As used herein, the "lipid" constituting the liposome is not particularly limited, as far as it comprises a hydrophilic group and a hydrophobic group, and is capable of constituting a vesicle. Examples include sterols such as cholesterol (Chol), phytosterol, 3β-[N-(dimethylaminoethane)carbamoyl]cholesterol (DC-Chol), N-(trimethylammonioethyl)carbamoyl cholesterol (TC-Chol); phosphatidylethanolamines such as dipalmitoyl phosphatidylethanolamine (DPPE), distearoyl phosphatidylethanolamine (DSPE); phosphatidylcholine such as dipalmitoyl phosphatidylcholine (DPPC), distearoyl phosphatidylcholine (DSPC); phosphatidylserine such as dipalmitoyl phosphatidylserine (DPPS), distearoyl phosphatidylserine (DSPS); phosphatidates such as dipalmitoyl phosphatidate (DPPA), distearoyl phosphatidiate (DSPA) or a combination of two or more of these lipids.

As used herein, "glycosylated lipid" is a lipid constituting the liposome of the present invention, and refers to a lipid conjugated with the sugar. Examples of the glycosylated lipid include mannobiosylated DPPE, mannotriosylated DPPE, mannotetraosylated DPPE, mannopentaosylated DPPE, mannohexaosylated DPPE or mannoheptaosylated DPPE, and a more preferable example is mannotriosylated DPPE. "Non-glycosylated lipid" is a lipid constituting the liposome of the present invention, and refers to a lipid conjugated with no sugar. "Non-glycosylated lipid" may be synonymous with the "lipid".

As used herein, "(the sugar-coated liposome) can induce and/or lead proliferation of an antigen-specific CTL independently of an MHC class II molecule" refers to ability to induce and/or to lead proliferation of the antigen-specific CTL in the presence of an MHC class I molecule-binding antigen and a sugar-coated liposome under the condition where MHC class II molecule-binding antigen is absent. A sugar-coated liposome can be confirmed to have ability to induce and /or to lead proliferation of an antigen-specific CTL without depending on an MHC class II molecule, by administering a sugar-coated liposome and an MHC class I molecule-binding antigen in the absence of an MHC class II molecule-binding antigen in vivo or in vitro, and by measuring the production of CTLs specific for the MHC class I molecule-binding antigen. More specifically, it can be confirmed, for example, according of the methods of Examples herein below.

## Advantageous Effects of Invention

The sugar-coated liposome of the present invention can activate CTLs without depending on MHC class II molecules. In particular, the sugar-coated liposome of the present invention encapsulating a tumor antigen specific for a MHC class I molecule can exhibit an antitumor effect without requiring a supportive stimulation. A sugar-coated liposome with the particle size of 100 to 1000 nm can increase the release of type 1 cytokine independent of MHC class II molecules. Similarly, the release of type 1 cytokine independently of MHC class II molecules can be increased by using the sugar-coated liposome of the present invention with the amount of coated sugar in a molar ratio of glycosylated lipid to non-glycosylated lipid be equal to or more than 0.00075.

## Brief Description of Drawings

[Figure 1] Figure 1 shows a result of the measurement of variation in the capacity of macrophage to produce IL-12p40 depending on the particle diameter. In the figure, the ordinate represents the concentration of IL-12p40, and the abscissa represents the average particle diameter of mannose-coated liposome.

[Figure 2] Figure 2 shows a result of the measurement of variation in the capacity of macrophage to produce IL-6 depending on the particle size. In the figure, the ordinate represents the concentration of IL-6, and the abscissa represents the average particle diameter of mannose-coated liposome.

[Figure 3] Figure 3 shows a result of the measurement of variation in the capacity of macrophage to produce IL-12p40 depending on the relative amount of mannose that coats the mannose-coated liposome. In the figure, the ordinate represents the concentration of IL-12p40, and the abscissa represents the relative amount of sugar (a molar ratio of glycosylated to non-glycosylated lipid) that coats Man3-DPPE.

[Figure 4] Figure 4 shows a result of the measurement of variation in the capacity of macrophage to produce IL-6 depending on the relative amount of mannose that coats the mannose-coated liposome. In the figure, the ordinate represents the concentration of IL-6, and the abscissa represents the relative amount of sugar (a molar ratio of glycosylated to non-glycosylated lipid) that coats Man3-DPPE.

[Figure 5] Figure 5 shows a result of the measurement of the capacity of a mannose-coated liposome in which a peptide (hereinafter, referred to as "$OVA_{257-264}$") consisting of 257th to the 264th of the amino acid sequence of ovalbumin is encapsulated, to induce antigen-specific CTLs. In the figure, the ordinate represents the cytotoxicity (%), and the abscissa represents the ratio of effector to target cells.

[Figure 6] Figure 6 shows a change in tumor size when survivin 2B80-88 peptide encapsulated in a mannose-coated liposome to model mice. In the figure, the abscissa represents weeks after starting the administration, and the ordinate represents the tumor size ($mm^3$).

[Figure 7] Figure 7 shows the correlation revealed by measuring the particle size of the prepared liposome with two particle size analyzers, one from Malvern Instruments Ltd. and the other from HORIBA, Ltd. In the graph, the ordinate represents a result measured with the particle size analyzer from HORIBA, Ltd., and the abscissa represents a result measured with that from Malvern Instruments Ltd.

[Figure 8] Figure 8 illustrates the procedure of measuring the capacity of MCLs with different particle sizes to activate macrophages in Example 8.

[Figure 9] Figure 9 shows a result of the measurement of the capacity of MCLs with different particle sizes to induce IL-12p40. In the graph, the ordinate represents the concentration (pg/mL) of IL-12p40, and the abscissa represents the particle size ($\mu$m) (the pore size of the membrane filter used in the extruder) of the liposome used.

[Figure 10] Figure 10 shows a result of the measurement of the capacity of MCLs with different particle sizes to induce IL-6. In the graph, the ordinate represents the concentration (ng/mL) of IL-12p40, the abscissa represents the particle size ($\mu$m) (the pore size of the membrane filter used in the extruder) of the liposome used.

[Figure 11] Figure 11 shows a fitted curve for the correlation between the particle size of MCL and the capacity to activate macrophages. The fitted curve was drawn with fitting, a graphing module of Excel. The ordinate represents the capacity to induce IL-12 (%), and the abscissa represents the particle size of the liposome measured by Zetasizer from Malvern Instruments Ltd.

Description of Embodiments

An MHC class I molecule-binding peptide can be produced using a method well known to those skilled in the art used for amino acid synthesis, for example, by chemical synthesis using the Fmoc or Boc methods. An MHC class I molecule-binding peptide can be also synthesizes using an automatic peptide synthesizer. Examples of such a peptide synthesizer include PSSM-8 (Shimadzu Corporation); Model 433A peptide synthesizer (Applied Biosystems, Inc.); and ACT 396 Apex (Advanced Chem Tech Inc.).

A nucleic acid encoding an MHC class I molecule-binding peptide can be prepared using a method well known to those skilled in the art used for nucleic acid synthesis. For example, a commercial nucleic acid synthesizer can be used, or a nucleic acid amplification such as PCR may be employed using a gene having a desired nucleotide sequence as a template and designing primers for amplifying the nucleic acid of interest, as appropriate.

"Sugar-coated liposome" can be produced using a lipid constituting the liposome as a raw material, and a method known as a method for producing an liposome in which an antigen is encapsulated, for example, a method disclosed in International Publication Nos. WO95/11704, WO2005/087196, WO2006/104199, WO2007/122756, or, Japanese Patent Laid-Open No. 2001-081044.

Specifically, a sugar-coated liposome can be obtained by preparing a conjugate (glycosylated lipid) of sugar and lipid (e.g., a conjugate of mannopentaose and DPPE or a conjugate of mannotriose and DPPE) in advance and producing a liposome from the conjugate by the following method. In particular, the liposome having a desired relative amount of sugar that coats the liposome according of the present invention can be synthesized by the following method of synthesizing a liposome using desired ratio of glycosylated and non-glycosylated lipids. A liposome can be prepared

by subjecting a lipid such as a phosphatide to a physical or chemical treatment, for example, the Bangham method (also referred to as the thin film method), reverse-phase evaporation, ultrasonic, extrusion, French press, homogenization, ethanol injection, dehydration-rehydration method. A liposome can be prepared by a method comprising the steps of heating a mixture containing one or more lipids, water, and a water-miscible organic solvent at a temperature range of 62 to 80°C to melt the lipid; and after the heating, cooling the mixture. The water-miscible organic solvent can be, for example, 5 to 30 vol. % to the total volume of the water and the water-miscible organic solvent.

Alternatively, a sugar-coated liposome can be obtained by introducing sugar to the surface of the liposome by mixing a liposome produced in the absence of glycosylated lipid and a glycosylated lipid and leaving it still at 4°C to room temperature for 24 to 120 hours. Alternatively, a sugar conjugated liposome can be also obtained by directly conjugating liposome and sugar.

The sugar-coated liposome having a desired particle size according of the present invention can be prepared by filtering through an appropriately selected filter or the like, so as to have a desired particle size or particle size distribution. For example, the liposome of the present invention may be a liposome sized through a 100 to 1000 nm filter, is preferably a liposome sized through a 200 to 1000 nm filter, and most preferably a liposome sized through a 600 to 1000 nm filter. The sugar-coated liposome having a desired particle size can be obtained by sizing a sugar-coated liposome ten times each using an extruder in sizing apparatus (Northern Lipids Inc.) and double filters (e.g., 100, 200, 400, 600, 1000 nm) with pressurizing in a range of 0.2 to 1 MPa.

When an MHC class I molecule-binding peptide is encapsulated in the sugar-coated liposome of the present invention, encapsulation of the peptide into sugar-coated liposome can be conducted by repeated freeze-thawing of liposomal suspension obtained by dissolving or suspending the peptide in a water-soluble solvent to be used for the liposomal production, or suspending the sugar-coated liposome into an aqueous solvent containing the peptide. The amount of the antigen peptide to be encapsulated in the sugar-coated liposome is usually 1 to 100 $\mu$g and more preferably 10 to 50 $\mu$g per mg of lipid constituting the sugar-coated liposome. The mannose-coated liposome in which the antigen peptide is encapsulated may be prepared by centrifugation or the like after being produced by a method described above, so that the concentration of the liposome would become higher in which the antigen is encapsulated.

Since the liposome of the present invention can activate CTLs independent of MHC class II molecules, the liposome can be used as a therapeutic or preventive drug (i.e. an animal medicine or a medicine) against various diseases in a mammal by appropriately selecting the MHC class I molecule-binding peptide or nucleic acid encoding the peptide to be used together. Examples of diseases that can be treated and/or prevented by the liposome of the present invention, or diseases that the pharmaceutical composition of the present invention is directed to treat and/or prevent include leukemia (minor histocompatibility antigens); tumor (tumor specific antigens); virus infections such as herpes simplex virus and influenza virus (virus antigens); parasitosis such as leishmania, toxoplasma, and malaria (parasite antigens); fungal infections such as candida, aspergillus, and cryptococcus (fungal antigens); and bacterial infections such as mycoplasma, and streptococcus (bacterial antigens). Examples of mammals that can be treated and/or prevented by the liposome of the invention include human, horse, sheep, pig, goat, cow, dog, cat, rabbit, mouse, and marmot. Accordingly, as used herein, pharmaceutical compositions include animal medicines as well as medicines for administration to human.

As used herein, "cancer" and "tumor" refer to mass of tissue proliferating autonomously, excessively, and invasively against the control in the body, and are synonymous with malignant tumor (including cancer and sarcoma) and malignant neoplasm. Examples of cancers or tumors that the sugar-coated liposome of the present invention and pharmaceutical composition are directed to treat and/or prevent include liver cancer, cholangioma, colon cancer, stomach cancer, breast cancer, pancreatic cancer, myeloma or leukemia.

When the sugar-coated liposome of the present invention is used as a medicine, examples of administration routes include oral administration, intraoral administration, intranasal administration, ophthalmic administration, tracheobronchial administration, subcutaneous administration, transdermal administration, transmucosal administration, intramuscular administration, and intravascular (intravenous) administration. Examples of formulations include an injection, a capsule, a tablet, a syrup, a granule, a patch, an ointment, a powder, and a spray. A medicine containing the sugar-coated liposome of the present invention as an active ingredient can be administered alone, or in combination with a pharmacologically acceptable carrier (see, "Japanese Pharmaceutical Excipients Directory", YAKUJI NIPPO LIMITED, and "Handbook of Pharmaceutical Excipients" American Public Health Association). When a medicine containing the sugar-coated liposome of the present invention as an active ingredient is used as an injection agent, examples of storage containers include ampoules, vials, prefilled syringes, pen type injection cartridges, and infusion bags.

The dosage of the pharmaceutical composition containing the sugar-coated liposome of the present invention as an active ingredient is not particularly limited as far as it provides a desired therapeutic or preventive effect, but is preferably subcutaneous or intramuscular administration. Choices of the dosage of the sugar-coated liposome of the present invention include an injection or various noninvasive administration methods such as oral, nasal, transdermal, and transmucosal administration. A pharmaceutical composition containing the sugar-coated liposome of the present invention as an active ingredient may be administered transiently and may administered continuously or intermittently.

For example, a dosage frequency of the sugar-coated liposome of the present invention is preferably a single administration and 1-4 times per week, and more preferably a single administration or the once a week. Occasional administration such as once every one to three months may be chosen. When the sugar-coated liposome and the MHC class I molecule-binding antigen of the present invention are administered in separate formulations, they can be administered separately at the same time, in continuous, or with an interval.

The dose of a medicine containing the sugar-coated liposome of the present invention as an active ingredient is not particularly limited as far as it provides a desired therapeutic or preventive effect, and can be determined appropriately depending on symptoms, sex, age and the like. The dose of the sugar-coated liposome of the present invention can be determined, for example, based on, as an indicator, a therapeutic or preventive effect of interest (or suppression of cancer metastasis in case the disease to be treated is cancer), or the number of cytotoxic T cells specific for the MHC class I molecule-binding peptide used in blood in the patient. For example, when the dose is determined on the basis of the weight of the subject, the liposome can be administered at a dose of 5 - 500 ng/kg (preferably 5 to 50 ng/kg, and more preferably 5 to 10 ng/kg) based on the amount of the antigen. When the dose is determined on the basis of head count, the liposome can be administered at a dose of 0.1 ng to 300 μg/head (preferably 0.1 ng to 50 μg/head, more preferably 0.2 ng to 10 μg/head) based on the amount of the antigen. For example, The dose of the medicine containing the sugar-coated liposome of the present invention as an active ingredient when administered to a human is 0.5 ng to 30 μg, more preferably 0.5 ng to 5 μg, further more preferably 1 ng to 1 μg per dose per patient based on the amount of the antigen.

Although the present invention will be hereinafter described in detail with reference to Examples, the present invention is not intended to be limited thereto.

Example 1

Preparation of Mannose-Coated Liposome (MCL)

(1) Preparation of MCL having different particle sizes

MCLs having different particle sizes were produced by the following method: 75.9mg of DPPC, 40 mg of cholesterol and 6.1mg of Man3-DPPE (DPPC : cholesterol : Man3-DPPE = 1:1:0.05 in the molar ratio) were mixed with 0.85mL of t-butanol solution in a glass vial. Lipid ingredients were dissolved in water bath at 70°C, and then 4mL of PBS solution was added to the dissolved lipid solution. The mixture was heated again in water bath at 70°C, and the lipid ingredients were dissolved. After the dissolving of the lipid, the mixture was stirred in water bath maintained at 70°C for ten minutes. MCLs were obtained by cooling with stirring at room temperature for 20 minutes. The resulted MCL suspensions were suspended into PBS, centrifuged, and separated from the supernatant for three times and t-butanol in the suspensions was removed. Subsequently, the MCLs were sized by filtering through double membrane filters (1000, 600, 400 and 200 nm in pore size) 10 times each in sequence using the extruder in a sizing apparatus (Northern Lipids Inc.) The respective MCL suspensions were washed and recovered by centrifuge, and then suspended into PBS. The obtained MCLs were subjected to measurement for the average particle diameter using a particle size analyzer (manufactured by Malvern Instruments Ltd., ZETA SIZER Nano-ZS) by dynamic light scattering. As a result, the average particle diameters of MCLs sized with 1000, 600, 400 and 200 nm in pore size were 596, 441, 308 and 178 nm, respectively. The analysis of the amount of cholesterol was carried out using a commercial kit, Cholesterol E-Test Wako (Wako Pure Chemical Industries Co., Ltd., 439-17501).

(2) Preparation of MCL having different amounts of mannotriose that coats liposome

Each of 18.3 mg, 6.1 mg, 1.83 mg, 0.61 mg, 0.183 mg, 0.061 mg, 0 mg of Man3-DPPE was added to 75.9 mg of DPPC and 40 mg of cholesterol (DPPC : cholesterol : Man3-DPPE (molar ratio) was 1:1:0.15, 1:1:0.05, 1:1:0.015, 1:1:0.005, 1:1:0.0015, 1:1:0.0005, 1:1:0). These mixtures were mixed with 0.85mL of t-butanol solution in glass vials. Lipid ingredients were dissolved in water bath at 70°C and then 4 mL of PBS was added to the lipid dissolved solution. The solution was heated again in water bath at 70°C and the lipid ingredients were dissolved. After the dissolving of the lipid, the mixture was stirred in water bath maintained at 70°C for ten minutes. MCLs were produced by cooling with stirring at room temperature for 20 minutes. The produced MCL suspensions were suspended into PBS, centrifuged, and separated from the supernatant for three times and t-butanol in the suspensions was removed. Subsequently, the produced MCLs were sized by filtering 10 times through double membrane filters with a pore size of 1000 nm with pressurized nitrogen gas using the extruder in a sizing apparatus (Northern Lipids Inc.) The respective MCL suspensions were washed and recovered by centrifuge, and then suspended into PBS. The obtained MCLs were subjected to a measurement for the average particle diameter using a particle size analyzer (manufactured by Malvern Instruments Ltd., ZETA SIZER Nano-ZS) by dynamic light scattering. As a result, the average particle diameters of MCLs prepared

at a molar ratio of DPPC : cholesterol : Man3-DPPE = 1:1:0.15 (0.075), 1:1:0.05 (0.025), 1:1:0.015 (0.0075), 1:1:0.005 (0.0025), 1:1:0.0015 (0.00075), 1:1:0.0005 (0.00025), 1:1:0 (0) (numbers in parenthesis are molars ratio of Man3-DPPE to non-glycosylated lipid) were 695, 609, 599, 554, 609, 529 and 588 nm, respectively. The analysis of the amount of cholesterol was carried out using a commercial kit, Cholesterol E-Test Wako (Wako Pure Chemical Industries Co., Ltd., 439-17501).

Example 2

Capacity of MCL Having Different Particle Sizes to Activate Macrophage

The abdomen of BALB/cCrSlc mice (Japan SLC, Inc.) at 8 weeks of age was disinfected with cotton soaked in 70% ethanol. The mice were then injected intraperitoneally with MCLs produced in Example 1 (1) having average particle diameters of 178, 308, 441 and 596 nm, in an amount corresponding to 60 μg of cholesterol, with syringes for tuberculin. One hour after the administration, mice were euthanized. 5mL of PBS was immediately injected into murine abdominal cavity and the abdominal cavity was washed and free cells in the abdominal cavity were recovered by recovering the washing solution from the abdominal cavity. The washing solution was centrifuged at 1,500 rpm for five minutes. Precipitated cells were suspended into 10 mL of RPMI medium containing 10% fetal bovine serum (FBS), and washed and centrifugal at 1,500 rpm for 5 minutes for several times. The cell suspensions were adjusted to 1 X $10^6$ cells/mL in RPMI medium containing 10% FBS and used to inoculate medium in cell culture dishes. The cells were cultured at 37°C in a 5% $CO_2$ incubator for 1 hour, and CD11b-positive cells (macrophages) were allowed to adhere, and then non-adhering cells were washed away. After medium change, the culture was continued at 37°C in a 5% $CO_2$ incubator for 20 hours and culture supernatant was recovered. Cytokines (IL-12p40 and IL-6) in the culture supernatant were measured using Mouse OptEIA ELISA kit (BD Biosciences).

The results of measurement of IL-12p40 and IL-6 are shown in Figure 1 and Figure 2, respectively. MCLs having an average particle diameter of 178 nm or more (especially, 308nm or more) have been revealed to have prominently increased production of IL-12 and reduced production of IL-6, and thereby have high capacities to induce immune responses in cell-mediated immunity.

Example 3

Capacity of MCL Having Different Amounts of Man3-DPPE That Coats Liposome to Activate Macrophage

The abdomen of BALB/cCrSlc mice (Japan SLC, Inc.) at 8 weeks of age was disinfected with cotton soaked in 70% ethanol. The mice were then injected intraperitoneally with MCLs prepared in Example 1 (2) having different relative amounts of Man3-DPPE (molar ratios of Man3-DPPE to non-glycosylated lipid (defined to be 1) were 0.075, 0.0025, 0.0075, 0.0025, 0.00075, 0.00025, 0, respectively) that coats the liposome, in an amount corresponding to 60 μg of cholesterol, with syringes for tuberculin. One hour after the administration, mice were euthanized. 5mL of PBS was immediately injected into murine abdominal cavity and the abdominal cavity was washed and free cells in the abdominal cavity were recovered by recovering the washing solution from the abdominal cavity. The washing solution was centrifuged at 1,500 rpm for five minutes. Precipitated cells were suspended into 10 mL of RPMI medium containing 10% FBS, and washed and centrifugal at 1,500 rpm for 5 minutes for several times. The cell suspensions were adjusted to 1 X $10^6$ cells/mL in RPMI medium containing 10% FBS and used to inoculate medium in cell culture dishes. The cells were cultured at 37°C in a 5% $CO_2$ incubator for 1 hour, and CD11b-positive cells (macrophages) were allowed to adhere, and then non-adhering cells were washed away. After medium change, the culture was continued at 37°C in a 5% $CO_2$ incubator for 20 hours and culture supernatant was recovered. Cytokines (IL-12p40 and IL-6) in the culture supernatant were measured by Mouse OptEIA ELISA kit (BD Biosciences).

The results of measurement of IL-12p40 and IL-6 are shown in Figure 3 and Figure 4, respectively. MCLs having a relative amount of Man3-DPPE that coats the liposome in a molar ratio to non-glycosylated lipid of 0.00075 or more (especially, 0.0075 or more) have been revealed to have prominently increased production of IL-12 and reduced production of IL-6, and thereby have high capacities to induce immune responses in cell-mediated immunity.

Example 4

Inductive Effect of Cytotoxicity of Mannose-Coated Liposome (MCL) in Which $OVA_{257-264}$ is Encapsulated

(1) Preparation of MCL in which antigenic peptide $OVA_{257-264}$ is encapsulated

75.9mg of DPPC, 40 mg of cholesterol and 6.1mg of Man3-DPPE (DPPC : cholesterol : Man3-DPPE = 1:1:0.05

in the molar ratio) were weight in glass vials, and mixed 0.85mL of t-butanol solution. Lipid ingredients were dissolved in water bath at 70°C and then 4mL of $OVA_{257-264}$ solution (0.5 mg/mL, 10 % sucrose) was added to the lipid dissolved solution. The solution was heated again in water bath at 70°C and the lipid ingredients were dissolved. After the dissolving of the lipid, the mixture was stirred in water bath maintained at 70°C for ten minutes. MCLs in which $OVA_{257-264}$ was encapsulated was produced by cooling with stirring at room temperature for 20 minutes.

The produced suspensions of MCL in which $OVA_{257-264}$ was encapsulated were suspended into PBS, centrifuged, and separated from the supernatant for three times, and t-butanol in the suspension and the antigen that had been not encapsulated in MCL were removed. Subsequently, the MCLs in which $OVA_{257-264}$ was encapsulated were sized by filtering 10 times through double membrane filters with a pore size of 1000 nm with pressurized nitrogen gas using the extruder in a sizing apparatus (Northern Lipids Inc.) The MCLs in which $OVA_{257-264}$ was encapsulated were washed and centrifuged, and then suspended in PBS. The analysis of obtained MCLs in which $OVA_{257-264}$ was encapsulated carried out using Cholesterol E-Test Wako (Wako Pure Chemical Industries Co., Ltd., 439-17501) for the amount of cholesterol and using HPLC for the amount of peptide encapsulated. The recovery of lipid was around 80%. The average particle diameter of MCL in which $OVA_{257-264}$ is encapsulated was 503nm.

(2) Cytotoxicity activity of MCL in which $OVA_{257-264}$ was encapsulated

The cytotoxicity activity of MCL in which $OVA_{257-264}$ was encapsulated was measured by the induction of CTL against mouse lymphoma E.G7-OVA. E.G7-OVA (ATCC, CRL-2113) is EL4 transfected with such a cDNA that cause stable and endogenous production of OVA/H2Kb complex that induces CTL activity. EL4 is thymoma cell line of the H-$2^b$ haplotype derived from C57BL/6 (see, M. W. Moore, et al., (1988) Cell, 54 (6): 777-785).

The solution of MCL in which $OVA_{257-264}$ is encapsulated containing 22.3 μg of $OVA_{257-264}$, and 4 mg of cholesterol in 1mL was used as a stock solution (1 time). 10 times, 100 times, 1000 times, and 10000 times dilutes of the stock solution with saline were prepared. Then, the equal amount of respective dilutes of MCL in which $OVA_{257-264}$ was encapsulated were measured such that the amounts of $OVA_{257-264}$ should be 96, 9.6, 0.96, and 0.096 ng. The total amount was adjusted to 100 μL with saline. The preparations of MCL in which $OVA_{257-264}$ was encapsulated containing the prepared amounts of $OVA_{257-264}$ were administered 100 μL/head, twice with a week interval, subcutaneously to the hips of three C57BL/6 mice (Japan SLC, Inc.) per groups. 17 days after the priming, the spleen was extracted from mice, and squashed with two pieces of slide glass, while soaking it in 5 mL of RPMI medium containing 10% FBS. Then, the splenocytes were transferred to a 15mL centrifugal tube and the hemolysis solution {140 mM $NH_4Cl$, 17 mM Tris-HCl (pH 7.65)} was added in the proportion of 1mL to all splenocytes extracted from one mouse. The mixture was treated on ice for one and half minutes to destroy hemocytes. After the treatment, an appropriate amount of RPMI medium containing 10% FBS was added, and lymphocytes were washed and centrifuged, and then isolated by removing the supernatant. $1 \times 10^7$ cells of the separated lymphocytes were cultured in 2 mL of RPMI medium containing 10% FBS and 1 μg of OVA257-264 at 37°C for 70 hours, which were used as effector cells. The effector cells (E) were mixed with that target cells (T) (E.G7-OVA cells: $10^4$ cells/well) in the following ratios (E/T Ratio = 50:1, 25:1, 12.5:1, 6.25:1, 3.125:1, 1:1), and then cultured at 37°C for four hours, measured cytotoxicity activity with CytoTox 96 assay kit (Promega) in the following procedure.

First, the effector cells were mixed at the following numbers ($50 \times 10^4$, $25 \times 10^4$, $12.5 \times 10^4$, $6.25 \times 10^4$, $3.125 \times 10^4$, $1 \times 10^4$ cells/50 μL, RPMI medium containing 5 % FBS) with the target cells (E.G7-OVA: $1 \times 10^4$ cells/50 μL, RPMI medium containing 5 % FBS) (cell number ratios were an effector cells : target cells = 50:1, 25:1, 12:1, 6:1, 3:1, 1:1). 100 μL aliquots of these cell suspensions were transferred into a roundbottom 96 well cell culture plate, centrifuged at 25°C, at 250 × g for four minutes, whereby both cells were brought in contact at the bottom of the plate and the reaction was promoted. The cell suspensions were cultured at 37°C for four hours and then centrifuged at 25°C, 250 × g for four minutes. 50 μL of the supernatant was mixed with 50 μL of substrate solution. After 30 minutes of the reaction, 50 μL of stop solution was added and the absorbance at 490nm was measured, the cytotoxicity activity was calculated according of the following equation.

[Expression]

$$\text{Cytotoxicity activity (\%)} = \{(\text{Experimental}) - (\text{Effecter Spontaneous}) - (\text{Target Spontaneous})\} / \{(\text{Target Maximum}) - (\text{Target Spontaneous})\} \times 100$$

Experimental: absorbance at 490nm of the sample in which target cells and effector cells have been mixed and reacted

Effecter Spontaneous: absorbance at 490nm of the sample in which only effector cells have been cultured
Target Spontaneous: absorbance at 490nm of the sample in which only target cells were cultured
Target Maximum: absorbance at 490nm of the sample in which all the target cells were lysed with the lysis solution

The results are shown in Figure 5. As a result of this experiment, the highest cytotoxicity (CTL) activity (65%) was observed with the amount of immune antigen in the MCL in which $OVA_{257-264}$ was encapsulated was 0.096 ng, Accordingly, the MCL was suggested to exhibit a strong adjuvant effect with a very small amount of antigen peptide.

Example 5

Preparation of Mannose-Coated Liposome Containing Peptide Consisting of 80th to 88th of Amino Acid Sequences of Survivin 2B (Hereinafter, Referred to as "SVN2B (80-88) - MCL")

(1) Preparation of SVN2B (80-88) -MCL

The peptide (SEQ ID NO 2) consisting of 80th to 88th of the amino acid sequence of survivin 2B (hereinafter, "SVN2B (80-88)") has been already reported to be an MHC class I molecule-binding antigen (Hirohashi, Y, Clinical Cancer Research (2002) 8:1731-1739). Accordingly, experiments were conducted on anticancer activity of SVN2B (80-88) alone or in combination with mannose-coated liposome. 75.9mg of DPPC, 40 mg of cholesterol and 6.1mg of Man3-DPPE (DPPC : cholesterol : Man3-DPPE = 1:1:0.05 in the molar ratio) were weight in glass vials, and mixed 0.85mL of t-butanol solution. Lipid ingredients were dissolved with stirring in water bath at 70°C, and then 4 ml of SVN2B (80-88) solution (1 mg/mL) (saline) was added to the lipid solution. The solution was heated again in water bath at 70°C and the lipid ingredients were dissolved. After the dissolution of the lipid, the mixture was stirred in water bath maintained at 70°C for ten minutes. SVN2B (80-88) -MCLs were prepared by cooling with stirring at room temperature for 20 minutes. The produced SVN2B (80-88) -MCL suspensions were suspended into PBS, centrifuged, and separated from the supernatant for three times, and t-butanol in the suspension and SVN2B (80-88) that had been not encapsulated in MCL were removed. Subsequently, SVN2B (80-88)-MCL was sized by filtering 10 times through double membrane filters with a pore size of 1000 nm with pressurized nitrogen gas using the extruder in a sizing apparatus (Northern Lipids Inc.) The average particle diameter was 441 nm. SVN2B (80-88)-MCL was then washed and centrifuged and suspended into PBS. The analysis of obtained SVN2B (80-88) -MCL was carried out for the amount of cholesterol using Cholesterol E-Test Wako (Wako Pure Chemical Industries Co., Ltd., 439-17501).

(2) Quantification of SVN2B (80-88) encapsulated in SVN2B (80-88) -MCL

The amount of SVN2B (80-88) encapsulated in mannose-coated liposome was measured according of the following procedures. In a 1.5mL tube, 400 μL methanol was added to 100 μL of SVN2B (80-88) -MCL suspension prepared in (1), and liposome was destroyed to release SVN2B (80-88). Released SVN2B (80-88) was measured using an HPLC system (Separations Module 2695-2489, Waters Corp.), a column (186002684, SunFire, C18 5 μm, 4.6 × 250 mm, Waters Corp.), and a guard column (186002684, SunFire, C18 5 μm, 4.6 × 20 mm, Waters Corp.); and water (0.1% TFA (208-02741, Wako Pure Chemical Industries. Ltd.)) and AcCN (0.1% TFA (208-02741, Wako Pure Chemical Industries. Ltd.)) as a mobile phase. The amount of SVN2B (80-88) was measured by the HPLC with an elution gradient and detection at a wavelength of 215nm. According of the quantification by HPLC, the amount of SVN2B (80-88) encapsulated was 16.6 μg per mg of cholesterol constituting the mannose-coated liposome.

Example 6

Anticancer Activity of SVN2B (80-88) -MCL

(1) Introduction of survivin2B into Renca cell

Survivin 2B was introduced into pIRES vector (Clontech, # 631605) to produce the pIRES-ss-survivin2B vector. Renca cells (ATCC # CRL-2947) were transfected with the produced pIRES-ss-survivin2B vector. The Renca cells were suspended in 2.5ml DMEM containing 10% FBS and mixed by pipetting, then seeded in 6-well plates and cultured at 37°C at 5% of $CO_2$. 10 μL of Lipofectamine 2000 was added to a 1.5mL tube, and suspended in 250 μL of Opti-MEM medium (Transfer reagent). 3 μL of pIRES-ss-survivin2B vector was added to a 1.5mL tube, and suspended in 250 μL of Opti-MEM medium (plasmid solution). After leaving still at room temperature for 5 minutes, the Transfer reagent and the plasmid solution were mixed. The mixture was left still at room temperature for 20 minutes. The mixture was added to the Renca cells being approximately 50% confluent. The Renca cells were cultured at 37°C at 5% $CO_2$. After two

days or later, the medium was changed into DMEM medium containing 10% FBS of FBS and 1 μg/mL of puromycin. The Renca cells were cultured at 37°C at 5% $CO_2$. Because the cells without the vector are killed on the second day after the medium change, the cells that proliferated thereafter were further cultured for seven days.

(2) Effect of SVN2B (80-88) -MCL on mouse transplanted with Renca cell expressing survivin 2B

BALB/C mice (Nippon Kurea Co., Ltd.) were transplanted subcutaneously at the back with Renca cells expressing survivin 2B (Renca-survivin cells) at $2 \times 10^5$ cells/animal. About two weeks later, the formation of tumors of 3 to 5 mm in diameter were confirmed, and SVN2B (80-88) -MCL produced in Example 5 was administered to mice. 200 uL of preparations containing 800ng, 166ng, 16.6ng, 1.66ng, 0.166ng of SVN2B (80-88) or (50 μg, 10 μg, 1 μg, 0.1 μg, 0.01 μg based on the amount of cholesterol) of SVN2B (80-88)-MCL per mouse and being suspended with PBS to 200 uL were administered. Empty MCL (50 μg based on the amount of cholesterol), a preparation in which 10 μg of SVN2B (80-88) was suspended in 200 μL of PBS and 200 μL of PBS were also administered as control, instead of SVN2B (80-88)-MCL. Each group consisted of 3 animals. The tumor volume was measured every week until the third week after the administration. Two animals in the 10 μg administrated group and one animal in 1 μg administrated group were killed before the measurement day on the fourth day.

(3) The results are shown in Figure 8. The highest effect of suppressing tumor growth was observed in the group administered with SVN2B (80-88)-MCL in which 1.66 ng of SVN2B (80-88) was encapsulated. Next to the group, the group administered with SVN2B (80-88) -MCL in which 0.166 ng of SVN2B (80-88) was encapsulated had high effects of suppressing tumor growth. The groups administered with empty MCL (50 μg based on the amount of cholesterol) and survivin2B-MCL in which 16.6 μg of SVN2B (80-88) was encapsulated showed comparable effects of suppressing tumor growth, which were positive effects of suppressing tumor growth in comparison with the negative control (the PBS administration group). The single administration of SVN2B (80-88) (10 μg) was not distinguishable with the PBS administrated group, and no effect of suppressing tumor growth was observed. Thus, while the MHC class I molecule-binding antigen SVN2B (80-88) was not capable of exhibiting an antitumor effect by itself even at 10 μg, MCL in which SVN2B (80-88) was encapsulated exhibited excellent antitumor effects in the absence of an MHC class II antigen even at very small amounts of 0.166 to 16.6 ng. As described above, amounts of vaccines used in conventional cancer immunotherapies are around 0.1-10 mg for humans and several tens of micrograms for mice. The MCL of the present invention exhibited an antitumor effect at very small amounts of antigen, which was 0.166 to 16.6 ng for mice (which correspond to 1ng to 1 μg of amounts of antigen for humans), in comparison with conventional techniques. It was shown that the MCL of the present invention is capable of suppressing tumor growth with ng order of antigen (SVN2B (80-88)) for mice and several nanograms to several hundred nanograms for humans.

Example 7

Preparation of Mannose-Coated Liposome (MCL) With Different Particle Sizes

MCLs were prepared using the same materials and method as Example 1, provided that total six membrane filters of 0.1, 0.2, 0.4, 0.6, 1.0, 2.0 μm in pore size were installed into the extruder, and the MCLs were filtered, sized and prepared for respective sizes. The particle size of the prepared liposomes was measured using two particle size analyzers, one from Malvern Instruments Ltd. and the other one from HORIBA, Ltd. The particle sizes measured with both measuring instruments had good parallelism, but HORIBA had more tendency of having higher values as particles get larger (Figure 7).

[Table 1]

| pore diameter of Extrude, average particle diameter (actual measured value) and induction ratio of each cytokine | | | | | |
|---|---|---|---|---|---|
| MCL sample No. | filter ϕ | Malvarn actual measured value average (μm,n=3) | HORIBA actual measured value (μm) | induction ratio of IL-6 | induction ratio of IL-12p40 (with reference to 0.1 μmϕ) |
| A | 2μϕ | 1.090 | 1.861 | 45% | 254% |
| B | 1μϕ | 0.508 | 0.998 | 31% | 368% |
| C | 0.6μϕ | 0.288 | 0.56 | 18% | 363% |
| D | 0.4μϕ | 0.219 | 0.315 | 17% | 133% |
| E | 0.2μϕ | 0.149 | 0.165 | 86% | 101% |

(continued)

| pore diameter of Extrude, average particle diameter (actual measured value) and induction ratio of each cytokine | | | | | |
|---|---|---|---|---|---|
| MCL sample No. | filter ϕ | Malvarn actual measured value average (μm,n=3) | HORIBA actual measured value (μm) | induction ratio of IL-6 | induction ratio of IL-12p40 (with reference to 0.1 μmϕ) |
| F | 0.1μϕ | 0.105 | 0.104 | 103% | 101% |

Example 8

Capacity of MCL Having Different Particle Sizes to Activate Macrophage

The capacity of MCLs having different particle sizes to activate macrophages was studied according of the procedure illustrated in Figure 8. Accordingly, the liposomes prepared in the Example 7 having different particle sizes were intraperitoneally administered to BALB/c mice (Japan SLC, Inc.) at 8 month of age. After euthanizing the mice one hour after the administration, 5mL of PBS was immediately injected and the abdominal cavity was washed and free cells therein were recovered by recovering the washing solution from the abdominal cavity. The recovered solution was centrifuged at 1500rpm for five minutes, and then precipitated cells were suspended in 10 ml of RPMI medium containing 10% FBS. After repeating the operation several times, the cells were seeded in culture dishes. The cells were cultured at 37°C in a 5% $CO_2$ incubator for one hour, and adherent cells (macrophages) were allowed to adhere, then non-adherent cells were washed away. After medium change, the cells were cultured at 37°C in a 5% $CO_2$ incubator for 20 hours, then the culture supernatant was recovered. IL-12p40 and IL-6 in the culture supernatant were measured using Mouse OptEIA ELISA kit (BD BioSciences).

The results of measurement of IL-12p40 and IL-6 are shown in Figure 9 and Figure 10, respectively. The capacity of MCL to induce IL-12p40 was dependent on the particle size, and the liposomes having particle sizes of 0.6 to 1.0 μm as measured with the apparatus from HORIBA (0.3 to 0.5 μm with Malvern) had the strongest effect of induction. The capacity to suppress IL-6 was strongest in the MCLs having particle sizes of 0.3 to 1.0 μm (0.2 to 0.5 μm with Malvern) as measured with the apparatus from HORIBA. The correlation between the particle size and reactivity was found to be of bell shaped.

A fitted curve for the correlation between the particle size of MCL and the capacity to activate macrophage was drawn and the range where the IL-12 induction rate is equal to or higher than 250 % of the base was calculated as the optimal rage of particle size for the cytokine induction. Accordingly, the curve of the correlation between the particle size of MCL and the capacity to activate macrophage was drawn using fitting, an Excel's graphing module (Figure 11). 250 % activated points in this curve were determined to be 0.25 to 1.1 μm (ϕ) by calculating the intersecting points using an approximate equation to link two points. For this calculation, the particle sizes as measured by the ZETA SIZER Nano-ZS from Malvern Instruments Ltd. were used.

## Claims

1. A mannose-coated liposome with a molar ratio of glycosylated to non-glycosylated lipid of 0.00075 or more and a particle size of 180 to 1100 nm for use in a method of treatment, wherein the method comprises administering the mannose-coated liposome and an MHC class I molecule-binding peptide.

2. The mannose-coated liposome for use of claim 1, wherein:

   - the MHC class I molecule-binding peptide is encapsulated in the mannose-coated liposome; or
   - the mannose-coated liposome is separately administered at the same time, at intervals, or continuously with the MHC class I molecule-binding peptide.

3. The mannose-coated liposome for use of claim 1 or 2, wherein:

   - said mannose-coated liposome can induce and/or lead proliferation of an antigen-specific CTL independently of an MHC class II molecule; and/or
   - the MHC class I molecule-binding peptide is an antigen which cannot induce and/or lead proliferation of an antigen-specific CTL in the absence of an MHC class II antigen.

4. The mannose-coated liposome for use of claim 1, wherein the MHC class I molecule-binding peptide is a tumor specific antigen, a soluble protein, a virus antigen, a protozoan antigen, a fungal antigen, or a bacteria antigen.

5. The mannose-coated liposome for use of claim 1, wherein the MHC class I molecule-binding peptide is a tumor antigen.

6. The mannose-coated liposome for use of claim 5, wherein the tumor antigen is a peptide antigen comprising an amino acid sequence from 80th to 88th of survivin 2B.

7. The mannose-coated liposome for use of any one of the preceding claims, wherein the molar ratio of glycosylated to non-glycosylated lipid is:

   - 0.00075 to 0.075;
   - 0.0075 or more; or
   - 0.0075 to 0.075.

8. The mannose-coated liposome for use of any one of the preceding claims, wherein the sugar is oligomannose.

9. The mannose-coated liposome for use of claim 8, wherein the oligomannose is mannobiose, mannotriose, mannotetraose, mannopentaose, mannohexaose or mannoheptaose.

10. A mannose-coated liposome, wherein:

- a particle size of the mannose-coated liposome is 180 to 1100nm; and
- a molar ratio of glycosylated to non-glycosylated lipid is:

  - 0.00075 or more;
  - 0.00075 to 0.075;
  - 0.0075 or more; or
  - 0.0075 to 0.075.

**11.** The mannose-coated liposome of claim 10, wherein the sugar is oligomannose.

**12.** The mannose-coated liposome of claim 11, wherein the oligomannose is mannobiose, mannotriose, mannotetraose, mannopentaose, mannohexaose or mannoheptaose.

**13.** A pharmaceutical composition comprising a liposome as defined in any one of claims 1 to 12 as an active ingredient, for use in a method of treatment which is an immunotherapy for cancer.

**14.** The pharmaceutical composition for use according to claim 13, wherein the pharmaceutical composition is administered at a dose of 0.5 ng to 30 μg per patient.

**Patentansprüche**

**1.** Mannose-beschichtetes Liposom mit einem Molverhältnis von glykosyliertem zu nicht glykosyliertem Lipid von 0,00075 oder mehr und einer Partikelgröße von 180 bis 1100 nm zur Verwendung in einem Behandlungsverfahren, wobei das Verfahren das Verabreichen des Mannose-beschichteten Liposoms und eines MHC-Klasse-I-Molekül-bindenden Peptids umfasst.

**2.** Mannose-beschichtetes Liposom zur Verwendung nach Anspruch 1, wobei:

- das MHC-Klasse-I-Molekül-bindende Peptid in dem Mannose-beschichteten Liposom verkapselt ist; oder
- das Mannose-beschichtete Liposom getrennt gleichzeitig, in Abständen oder kontinuierlich mit dem MHC-Klasse-I-Molekül-bindenden Peptid verabreicht wird.

**3.** Mannose-beschichtetes Liposom zur Verwendung nach Anspruch 1 oder 2, wobei:

- das Mannose-beschichtete Liposom eine Proliferation einer antigenspezifischen CTL unabhängig von einem MHC-Klasse-II-Molekül induzieren und/oder lenken kann; und/oder
- das MHC-Klasse-I-Molekül-bindende Peptid ein Antigen ist, das eine Proliferation einer antigenspezifischen CTL in Abwesenheit eines MHC-Klasse-II-Antigens nicht induzieren und/oder lenken kann.

**4.** Mannose-beschichtetes Liposom zur Verwendung nach Anspruch 1, wobei das MHC-Klasse-I-Molekül-bindende Peptid ein tumorspezifisches Antigen, ein lösliches Protein, ein Virus-Antigen, ein Protozooen-Antigen, ein Pilz-Antigen oder ein Bakterien-Antigen ist.

**5.** Mannose-beschichtetes Liposom zur Verwendung nach Anspruch 1, wobei das MHC-Klasse-I-Molekül-bindende Peptid ein Tumorantigen ist.

**6.** Mannose-beschichtetes Liposom zur Verwendung nach Anspruch 5, wobei das Tumorantigen ein Peptidantigen ist, das eine Aminosäuresequenz der Positionen 80 bis 88 von Survivin B umfasst.

**7.** Mannose-beschichtetes Liposom zur Verwendung nach einem der vorhergehenden Ansprüche, wobei das Molverhältnis von glykosyliertem zu nicht glykosyliertem Lipid Folgendes ist:

- 0,00075 bis 0,075;
- 0,0075 oder mehr; oder
- 0,0075 bis 0,075.

**8.** Mannose-beschichtetes Liposom zur Verwendung nach einem der vorhergehenden Ansprüche, wobei der Zucker

Oligomannose ist.

**9.** Mannose-beschichtetes Liposom zur Verwendung nach Anspruch 8, wobei die Oligomannose Mannobiose, Mannotriose, Mannotetraose, Mannopentaose, Mannohexaose oder Mannoheptaose ist.

**10.** Mannose-beschichtetes Liposom, wobei

- eine Partikelgröße des Mannose-beschichteten Liposoms 180 bis 1100 nm ist; und
- ein Molverhältnis von glykosyliertem zu nicht glykosyliertem Lipid Folgendes ist:
    - 0,00075 oder mehr;
    - 0,00075 bis 0,075
    - 0,0075 oder mehr; oder
    - 0,0075 bis 0,075.

**11.** Mannose-beschichtetes Liposom nach Anspruch 10, wobei der Zucker Oligomannose ist.

**12.** Mannose-beschichtetes Liposom nach Anspruch 11, wobei die Oligomannose Mannobiose, Mannotriose, Mannotetraose, Mannopentaose, Mannohexaose oder Mannoheptaose ist.

**13.** Pharmazeutische Zusammensetzung, umfassend ein Liposom, wie in einem der Ansprüche 1 bis 12 definiert, als einen Wirkstoff, zur Verwendung in einem Behandlungsverfahren, das eine Immuntherapie für Krebs ist.

**14.** Pharmazeutische Zusammensetzung zur Verwendung nach Anspruch 13, wobei die pharmazeutische Zusammensetzung in einer Dosis von 0,5 ng bis 30 $\mu$g je Patient verabreicht wird.

**Revendications**

**1.** Liposome enrobé de mannose avec un rapport molaire de lipide glycosylé à non glycosylé de 0,00075 ou plus et une taille particulaire de 180 à 1100 nm destiné à une utilisation dans une méthode de traitement, la méthode comprenant l’administration du liposome enrobé de mannose et d’un peptide de liaison à une molécule de classe I MHC.

**2.** Liposome enrobé de mannose destiné à une utilisation selon la revendication 1, dans lequel :

- le peptide de liaison à une molécule de classe I MHC est encapsulé dans le liposome enrobé de mannose ; ou
- le liposome enrobé de mannose est administré séparément en même temps, par intervalles, ou de manière continue avec le peptide de liaison à une molécule de classe I MHC.

**3.** Liposome enrobé de mannose destiné à une utilisation selon la revendication 1, dans lequel :

- ledit liposome enrobé de mannose peut induire et/ou conduire à la prolifération d’un lymphocyte T cytotoxique (CTL) spécifique à un antigène indépendamment d’une molécule de classe II MHC ; et/ou
- le peptide de liaison à une molécule de classe I MHC est un antigène qui ne peut pas induire et/ou conduire à une prolifération d’un CTL spécifique à un antigène en l’absence d’un antigène de classe II MHC.

**4.** Liposome enrobé de mannose destiné à une utilisation selon la revendication 1, dans lequel le peptide de liaison à une molécule de classe I MHC est un antigène spécifique de tumeur, une protéine soluble, un antigène viral, un antigène protozoaire, un antigène fongique ou un antigène de bactérie.

**5.** Liposome enrobé de mannose destiné à une utilisation selon la revendication 1, dans lequel le peptide de liaison à une molécule de classe I MHC est un antigène tumoral.

**6.** Liposome enrobé de mannose destiné à une utilisation selon la revendication 5, dans lequel l’antigène tumoral est un antigène de peptide comprenant une séquence d’acides aminés du 80$^{ème}$ au 88$^{ème}$ de la survivine 2B.

**7.** Liposome enrobé de mannose destiné à une utilisation selon l’une quelconque des revendications précédentes,

dans lequel le rapport molaire de lipide glycosylé à non glycosylé est :

- 0,00075 à 0,075 ;
- 0,0075 ou plus ; ou
- 0,0075 à 0,075.

**8.** Liposome enrobé de mannose destiné à une utilisation selon l'une quelconque des revendications précédentes, dans lequel le sucre est l'oligomannose.

**9.** Liposome enrobé de mannose destiné à une utilisation selon la revendication 8, dans lequel l'oligomannose est le mannobiose, le mannotriose, le mannotétraose, le mannopentaose, le mannohexaose et le mannoheptaose.

**10.** Liposome enrobé de mannose, dans lequel :

- une taille particulaire du liposome enrobé de mannose est de 180 à 1100 nm ; et
- un rapport molaire de lipide glycosylé à non glycosylé est :
  - 0,00075 ou plus ;
  - 0,00075 à 0,075 ;
  - 0,0075 ou plus ; ou
  - 0,0075 à 0,075.

**11.** Liposome enrobé de mannose selon la revendication 10, dans lequel le sucre est l'oligomannose.

**12.** Liposome enrobé de mannose selon la revendication 11, dans lequel l'oligomannose est le mannobiose, le mannotriose, le mannotétraose, le mannopentaose, le mannohexaose et le mannoheptaose.

**13.** Composition pharmaceutique comprenant un liposome tel que défini dans l'une quelconque des revendications 1 à 12 en tant qu'ingrédient actif, destinée à une utilisation dans une méthode de traitement qui est une immunothérapie pour le cancer.

**14.** Composition pharmaceutique destinée à une utilisation selon la revendication 13, la composition pharmaceutique étant administrée à une dose de 0,5 ng à 30 μg par patient.

Fig.1

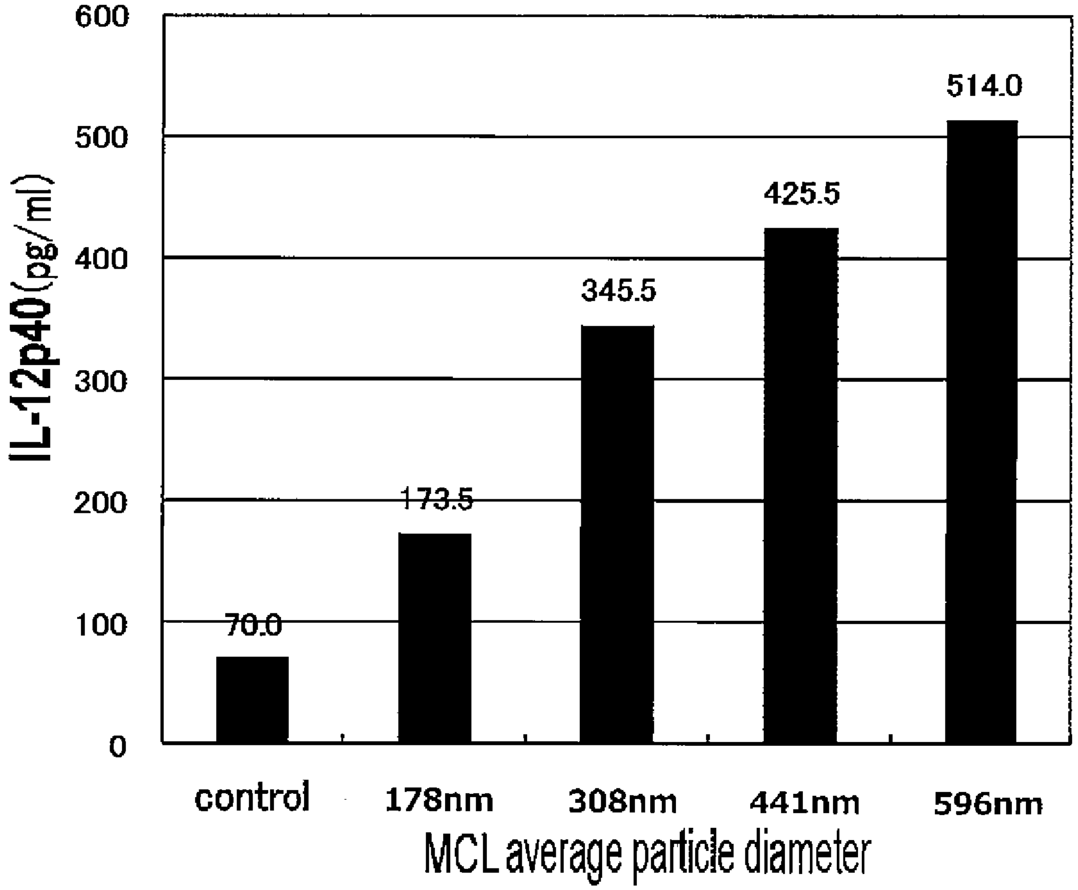
600
500
400
300
200
100
0
IL-12p40(pg/ml)
514.0
425.5
345.5
173.5
70.0
control
178nm
308nm
441nm
596nm
MCL average particle diameter

Fig.2

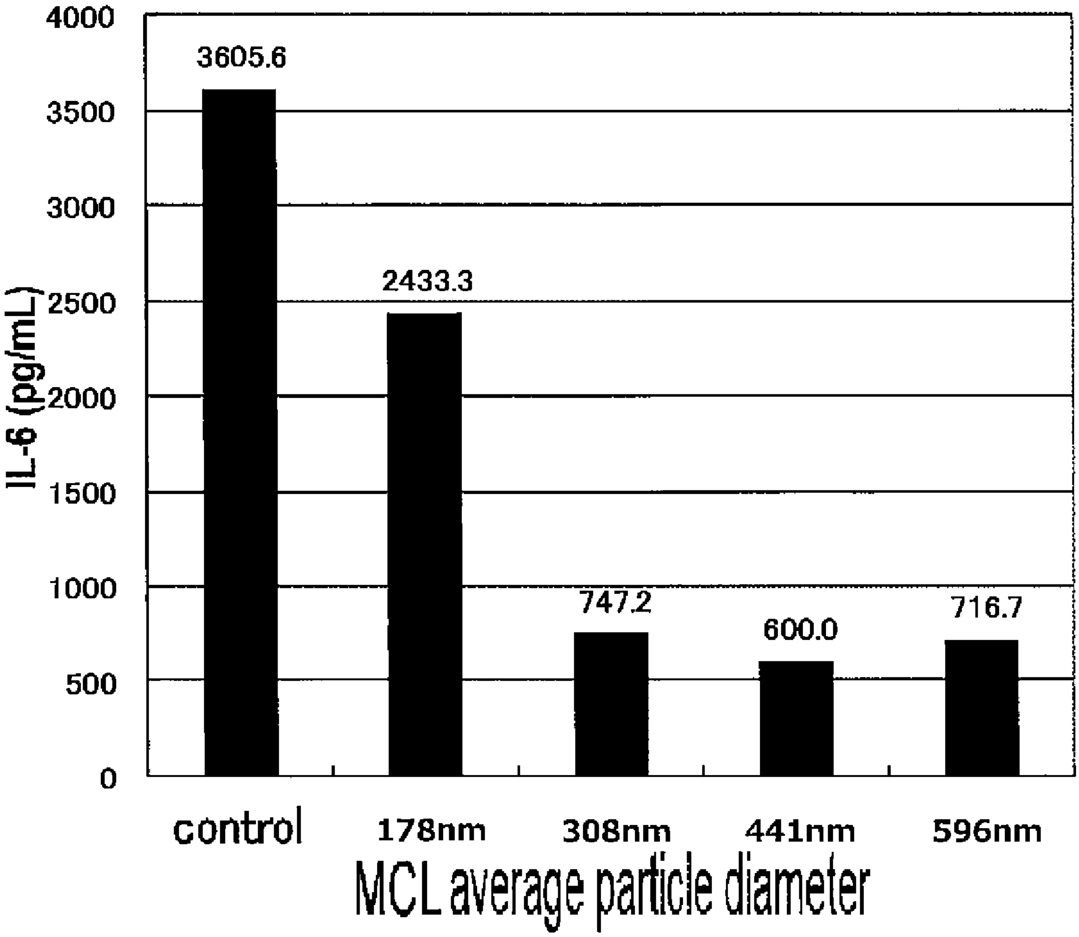
4000
3500
3000
2500
2000
1500
1000
500
0
IL-6 (pg/mL)
3605.6
2433.3
747.2
600.0
716.7
control
178nm
308nm
441nm
596nm
MCL average particle diameter

Fig.3

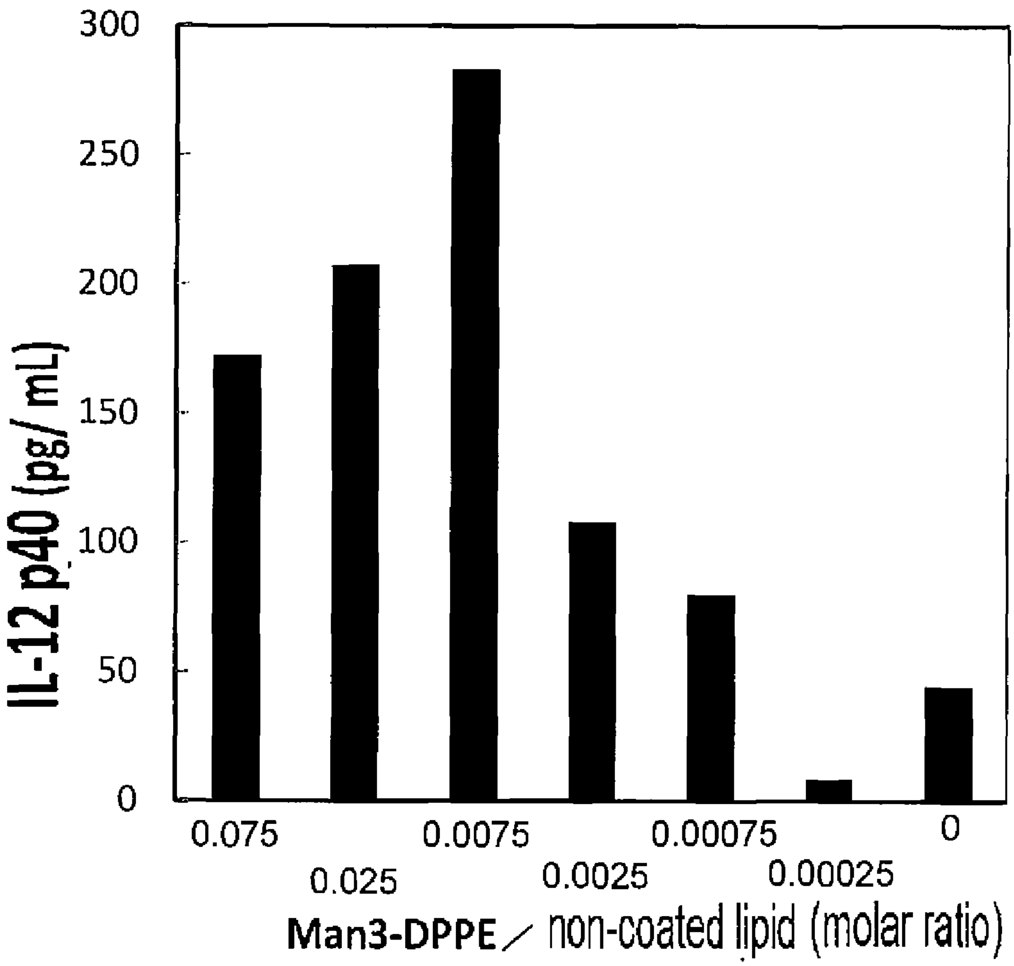
300
250
200
150
100
50
0
IL-12 p40 (pg/ mL)
0.075
0.025
0.0075
0.0025
0.00075
0.00025
0
Man3-DPPE／ non-coated lipid (molar ratio)

Fig.4

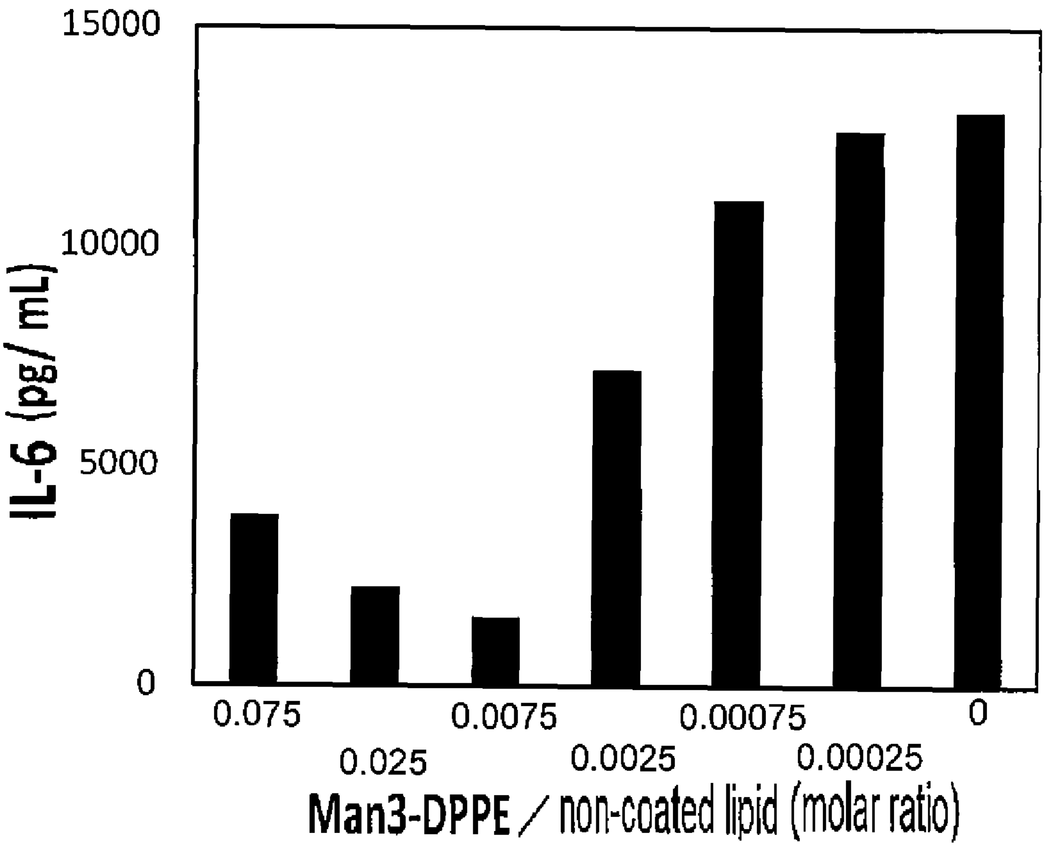
15000
10000
5000
0
IL-6 (pg/ mL)
0.075
0.025
0.0075
0.0025
0.00075
0.00025
0
Man3-DPPE / non-coated lipid (molar ratio)

Fig.5

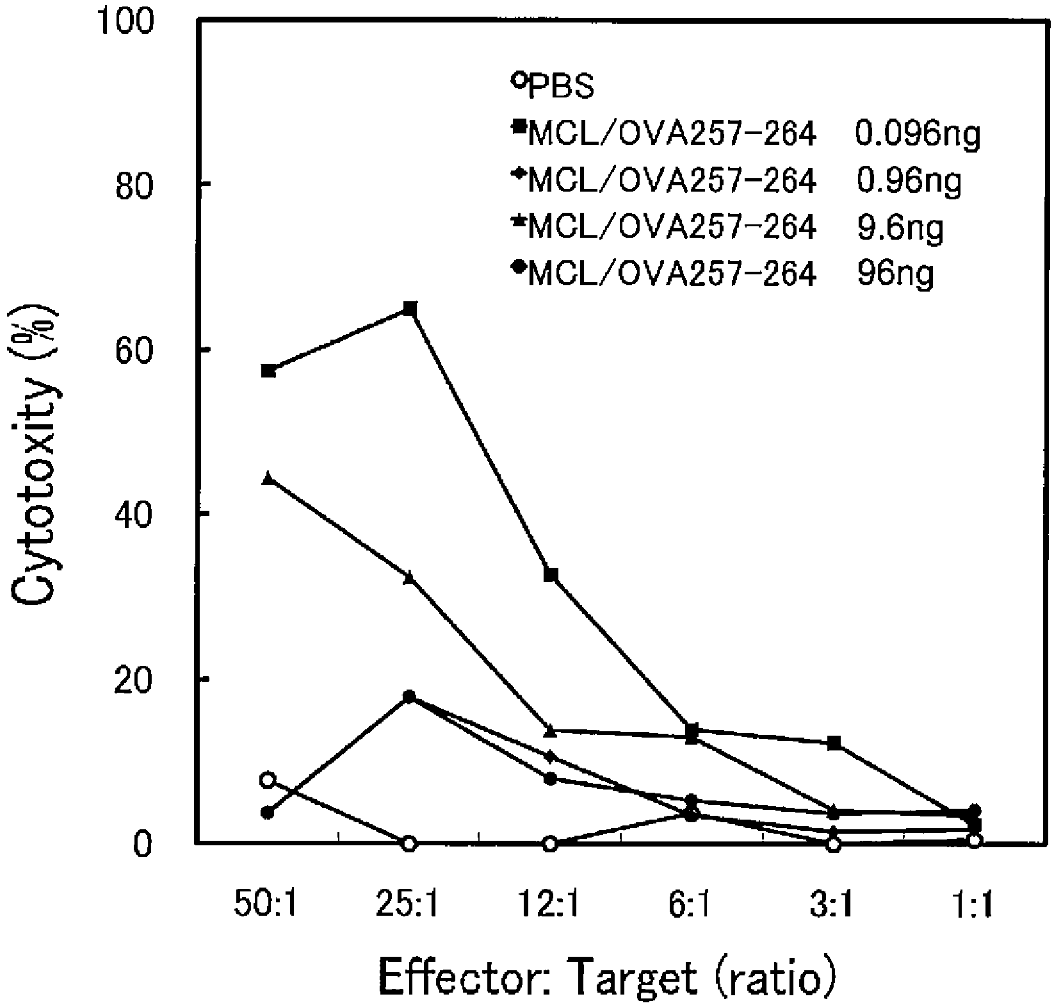
100
80
60
40
20
0
Cytotoxity (%)
PBS
MCL/OVA257-264 0.096ng
MCL/OVA257-264 0.96ng
MCL/OVA257-264 9.6ng
MCL/OVA257-264 96ng
50:1
25:1
12:1
6:1
3:1
1:1
Effector: Target (ratio)

Fig.6

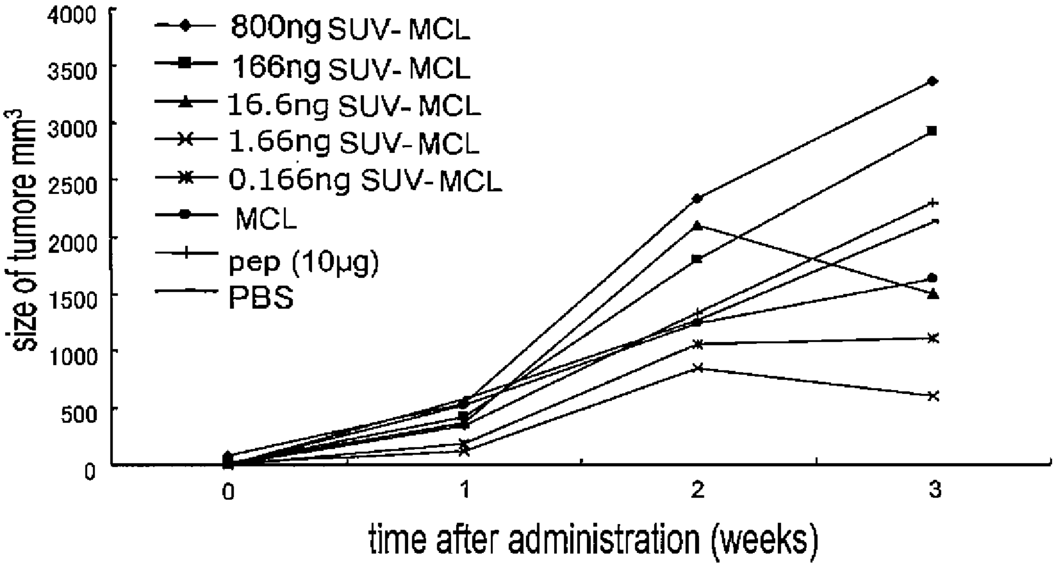
4000
3500
3000
2500
2000
1500
1000
500
0
size of tumore mm³
800ng SUV- MCL
166ng SUV- MCL
16.6ng SUV- MCL
1.66ng SUV- MCL
0.166ng SUV- MCL
MCL
pep (10µg)
PBS
0
1
2
3
time after administration (weeks)

Fig.7

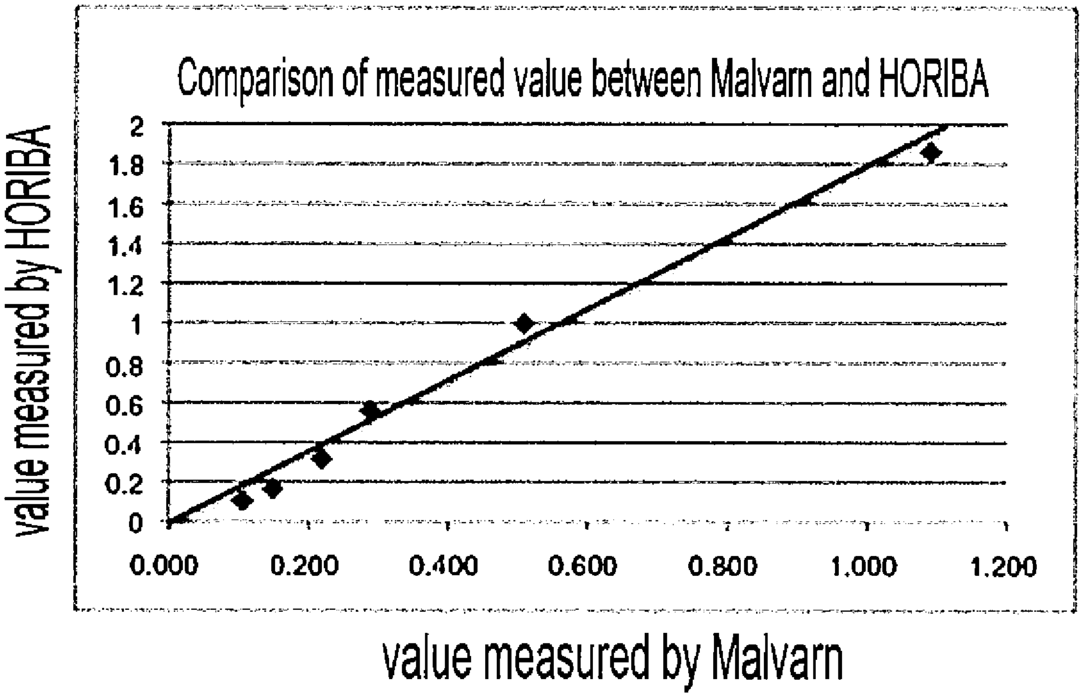
Comparison of measured value between Malvarn and HORIBA
value measured by HORIBA
2
1.8
1.6
1.4
1.2
1
0.8
0.6
0.4
0.2
0
0.000
0.200
0.400
0.600
0.800
1.000
1.200
value measured by Malvarn

Fig.8

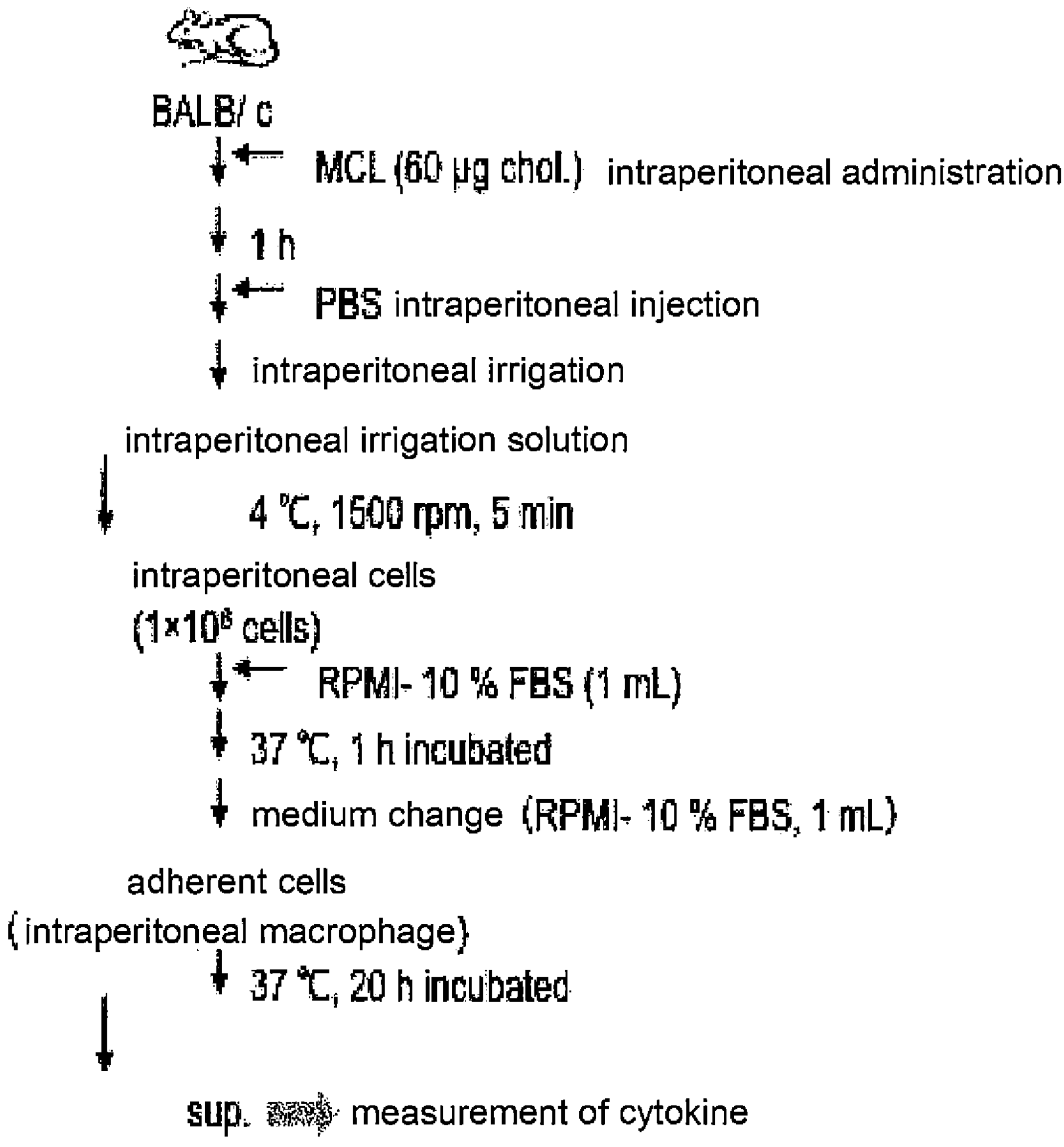
BALB/c
MCL (60 μg chol.) intraperitoneal administration
1 h
PBS intraperitoneal injection
intraperitoneal irrigation
intraperitoneal irrigation solution
4 °C, 1500 rpm, 5 min
intraperitoneal cells
(1×10^6 cells)
RPMI- 10 % FBS (1 mL)
37 °C, 1 h incubated
medium change (RPMI- 10 % FBS, 1 mL)
adherent cells
(intraperitoneal macrophage)
37 °C, 20 h incubated
sup.
measurement of cytokine

Fig.9

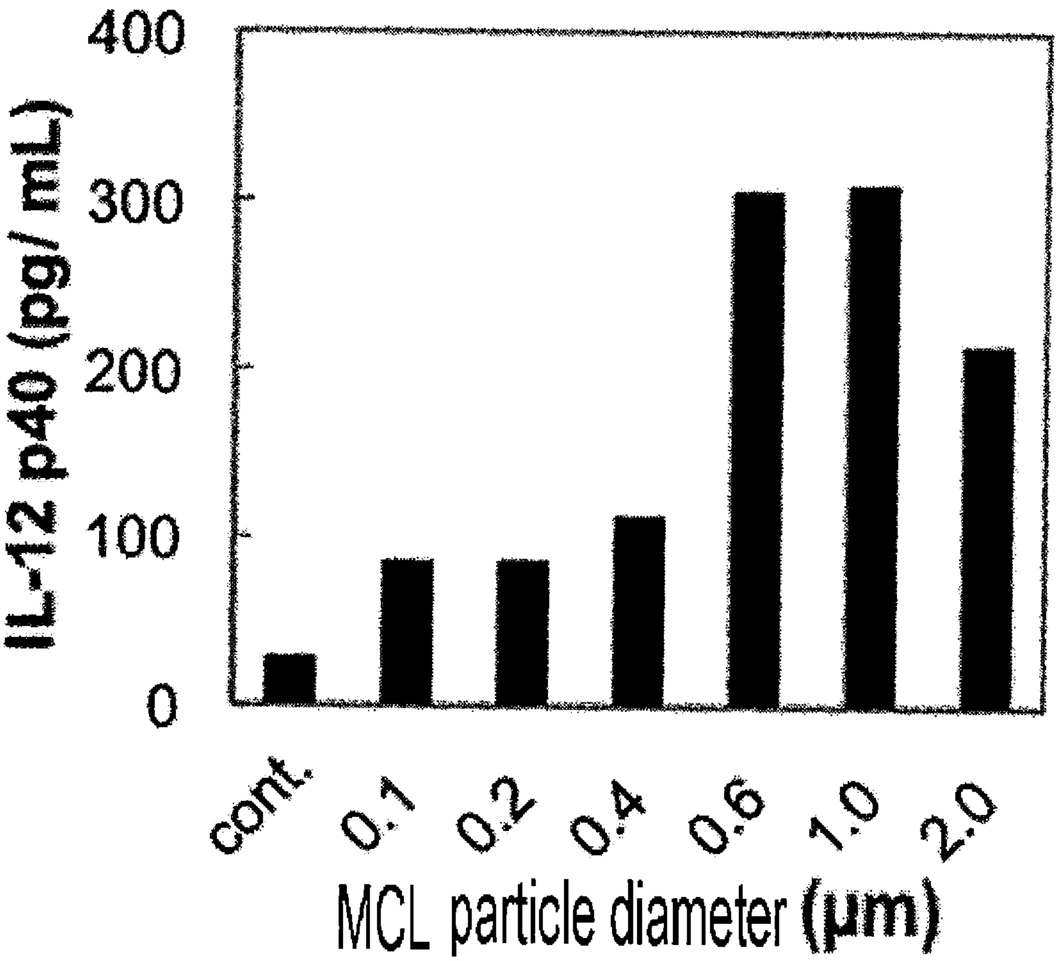
400
300
200
100
0
IL-12 p40 (pg/ mL)
cont.
0.1
0.2
0.4
0.6
1.0
2.0
MCL particle diameter (µm)

Fig.10

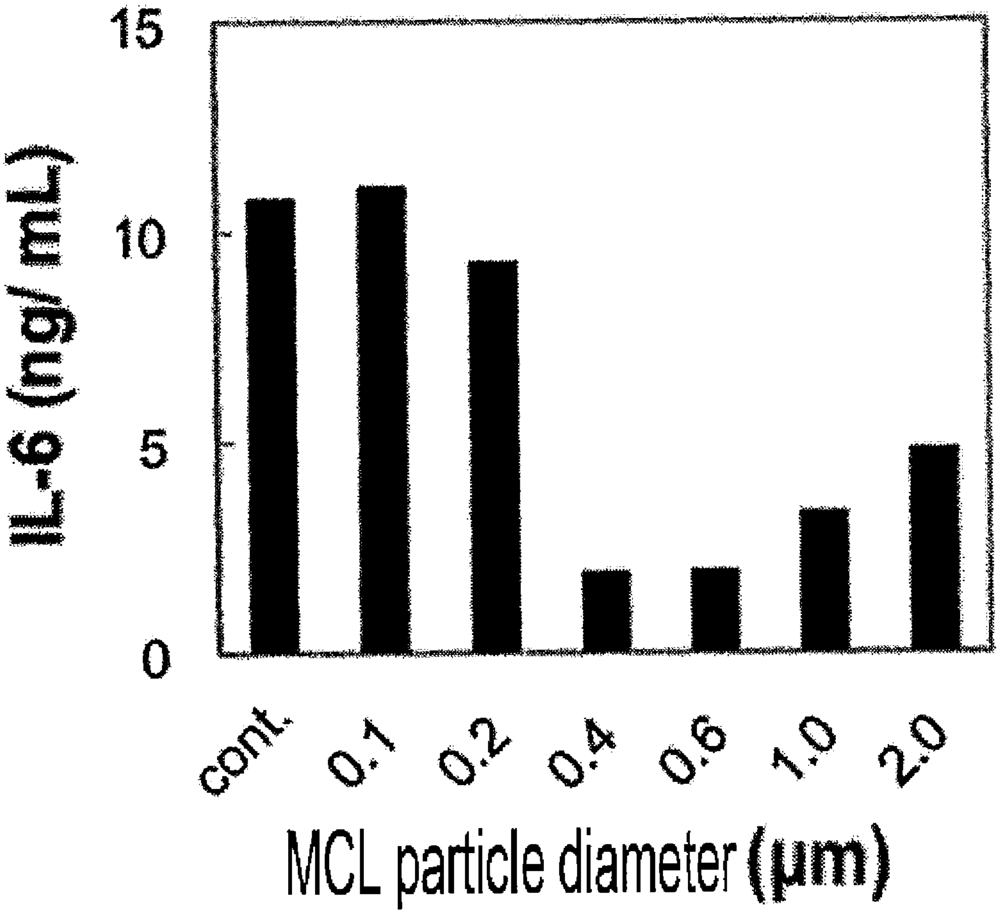
IL-6 (ng/ mL)
15
10
5
0
cont.
0.1
0.2
0.4
0.6
1.0
2.0
MCL particle diameter (µm)

Fig.11

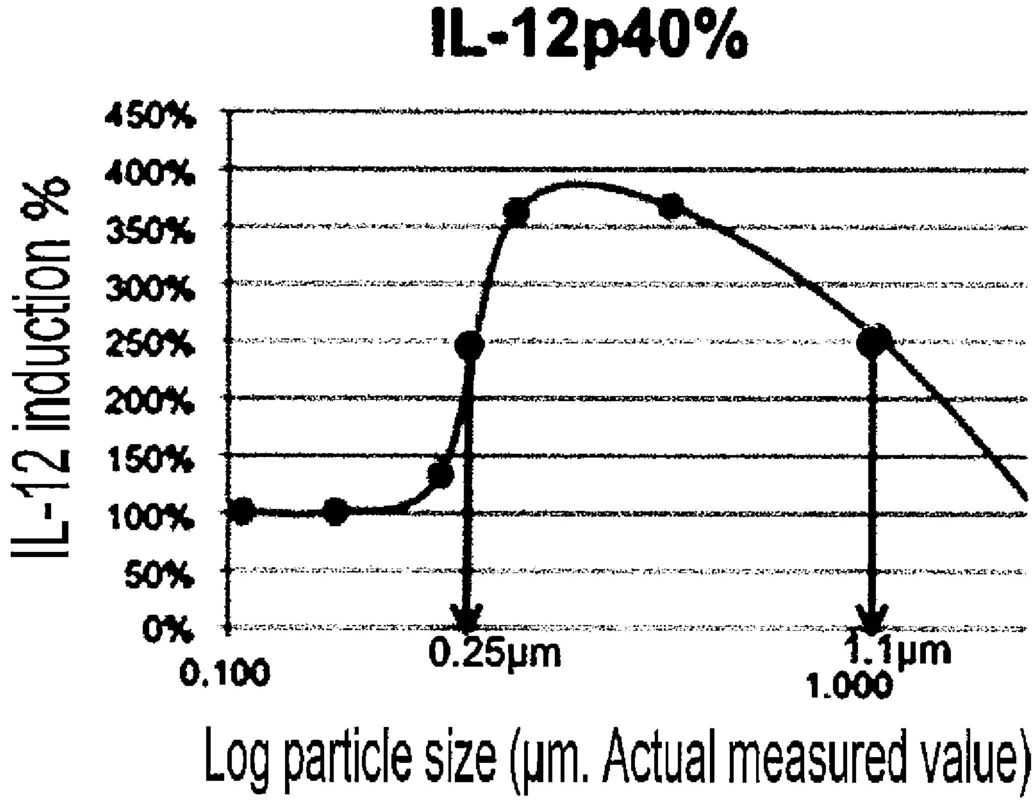
IL-12p40%
IL-12 induction %
450%
400%
350%
300%
250%
200%
150%
100%
50%
0%
0.100
0.25µm
1.1µm
1.000
Log particle size (µm. Actual measured value)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7126185 A
- WO 2006104199 A
- WO 9511704 A
- WO 2005087196 A
- WO 2007122756 A
- JP 2001081044 A
- JP 2011102962 A


### Non-patent literature cited in the description


- **IWAYAMA et al.** Surgical Treatment. *Gekachiryo,* 2007, vol. 96, 50-54
- **TAKASHI NISHIMURA.** Proceedings in Medicine. *Igakunoayumi,* vol. 221 (8), 641-646
- **SANDRA HERVAS-STUBBS et al.** *BLOOD,* 2007, vol. 109, 5318-5326
- **IKEDA et al.** *Experimental Medicine,* 2009, vol. 27 (14), 2176-2184
- **TSURUMA TETSUHIRO et al.** *Japanese Journal of Cancer and Chemotherapy,* 2004, vol. 31 (11), 1634-1636
- **ROSENBERG, S.A. et al.** *Nature Medicine,* 2004, vol. 10, 909-915
- **OKA et al.** *Japanese Journal of Clinical Immunology,* 2008, vol. 31 (5), 375-382
- **TSUBOI, A et al.** *Int. J. Hematol.,* 2007, vol. 86, 414-417
- **KUROTANI et al.** *The Journal of Immunology,* vol. 179, 1803-1813
- **LAKSHMI KRISHNAN et al.** *Cancer Research,* 2003, vol. 63, 2526-2534
- **SANGHA R. et al.** *Clin. Cancer Res.,* 2007, vol. 13 (15), 4652-4654
- **FUKUSAWA et al.** *FEBS Letters,* 1998, vol. 441, 353-356
- **TAKAGI et al.** *Cytokine,* 2007, vol. 40, 241-250
- **Y. SHIMIZU et al.** *Parasite Immunology,* 2007, vol. 29, 229-239
- **NAOYA KOJIMA et al.** *Journal of Controlled Release,* 2008, vol. 129, 26-32
- **Y. SHIMIZU et al.** *Parasite Immunology,* vol. 29
- **YUZURU IKEHARA et al.** *Cancer Res.,* 2006, vol. 66 (17), 8740-8748
- **SHIMIZU, Y., J. et al.** *Chromatogr. B.,* 2001, vol. 754, 127-133
- **M. W. MOORE et al.** *Cell,* 1988, vol. 54 (6), 777-785
- **HIROHASHI, Y.** *Clinical Cancer Research,* 2002, vol. 8, 1731-1739